(19) Europäisches Patentamt
European Patent Office
Office européen des brevets

(11) Publication number: **0 133 259 A2**

(12) **EUROPEAN PATENT APPLICATION**

(21) Application number: **84108608.5**

(22) Date of filing: **20.07.84**

(51) Int. Cl.[4]: **C 07 C 103/50**, C 07 C 87/22, C 07 C 102/00, C 07 C 85/00, A 61 K 31/16, A 61 K 31/13

(30) Priority: **21.07.83 JP 131845/83**

(43) Date of publication of application: **20.02.85 Bulletin 85/8**

(84) Designated Contracting States: **BE CH DE FR GB IT LI NL SE**

(71) Applicant: **KOWA COMPANY, LTD., 6-29, 3-chome Nishiki, Naka-ku Nagoya-shi Aichi-ken (JP)**

(72) Inventor: **Uchida, Yasumi, 3-1739, Ohno, Ichikawa-shi Chiba-ken (JP)**
Inventor: **Shigyo, Hiromichi, 6-18-8, Yotsuya, Fuchu-shi Tokyo (JP)**
Inventor: **Sato, Seiichi, 1-6-77, Honcho Higashi-murayama-shi, Tokyo (JP)**
Inventor: **Shibuya, Kimiyuki, 2-17-43, Noguchi-cho, Higashimurayama-shi Tokyo (JP)**
Inventor: **Ohta, Tomio, 4-41, Unoki Sayama-shi, Saitama-ken (JP)**
Inventor: **Ishihama, Hiroshi, 2-8-8, Suwa-cho, Higashi-murayama-shi Tokyo (JP)**

(74) Representative: **Kraus, Walter, Dr. et al, Patentanwälte Kraus, Weisert & Partner Irmgardstrasse 15, D-8000 München 71 (DE)**

(54) **Aminoalkyl-substituted benzene derivatives, process for production thereof, and pharmaceutical composition thereof.**

(57) An animoalkyl-substituted benzene derivative represented by the formula

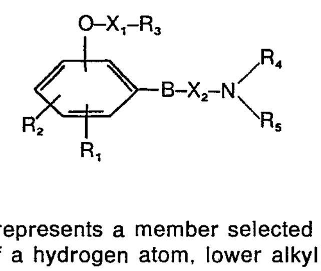

.....(I)

wherein $R_1$ represents a member selected from the class consisting of a hydrogen atom, lower alkyl groups, lower alkoxy groups, lower aliphatic acyl groups, halogen atoms and a trifluoroethoxy group; $R_2$ represents a hydrogen atom or a lower alkyl group; $R_3$ represents $-ONO_2$, $-CF_3$ or F; $R_4$ and $R_5$, independently from each other, represent a member selected from the class consisting of a hydrogen atom, lower alkyl groups, halogen-substituted benzoyl groups, phenyl-substituted lower aliphatic acyl groups and a pyridinecarbonyl group, or $R_4$ and $R_5$ may form, together with the nitrogen atom to which they are bonded, a 6-membered hetero ring which may contain another hetero atom selected from O and N; $X_1$ and $X_2$, independently from each other, represents an alkylene group having 1 to 8 carbon atoms; and B represents -O- or -NHCO- in which the nitrogen atom of -NHCO- is bonded to a carbon atom of the benzene ring; and an acid addition salt thereof; a process for production thereof; and pharmaceutical composition thereof.

EP 0 133 259 A2

This invention relates to aminoalkyl-substituted benzene derivatives which have useful pharmacological activity as drugs for treating diseases of the circulatory system, particularly arrhythmia, and which are not described in the prior literature; a process for production thereof; and a pharmaceutical composition thereof.

More specifically, this invention relates to aminoalkyl-substituted benzene derivatives represented by the following formula (I)

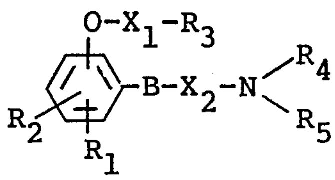

.....(I)

wherein $R_1$ represents a member selected from the class consisting of a hydrogen atom, lower alkyl groups, lower alkoxy groups, lower aliphatic acyl groups, halogen atoms and a trifluoroethoxy group; $R_2$ represents a hydrogen atom or a lower alkyl group; $R_3$ represents $-ONO_2$, $-CF_3$ or F; $R_4$ and $R_5$, independently from each other, represent a member selected from the class consisting of a hydrogen atom, lower alkyl groups, halogen-substituted benzoyl groups, phenyl-substituted lower aliphatic acyl groups and a pyridinecarbonyl group, or $R_4$ and $R_5$ may form, together with the nitrogen atom to which they are bonded, a 6-membered hetero ring which may contain another hetero atom selected from O and N; $X_1$ and $X_2$, independently from each other, represents an alkylene group having 1 to 8 carbon atoms; and B represents -O- or -NHCO- in which the nitrogen atom of -NHCO- is bonded to a carbon atom of the benzene ring; and acid addition salts thereof.

This invention also relates to a process for producing the compounds of formula (I) and the acid addition

salts thereof, and a pharmaceutical composition useful for treating diseases of the circulatory system, particularly arrhythmia comprising at least one of the compounds of formula (I) and the pharmaceutically acceptable acid addition salts thereof.

Analogous compounds having antiarrhythmic activity have previously been known. For example, West German Patent No. 2,235,745 discloses "Tocainide" represented by the following formula.

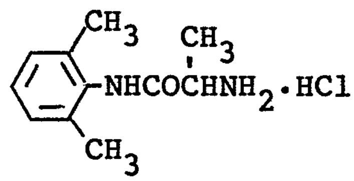

U. S. Patent No. 2,441,498 discloses "Lidocaine" represented by the following formula.

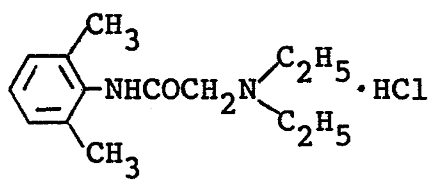

U. S. Patent No. 3,659,019 discloses "Mexiletine" of the following formula.

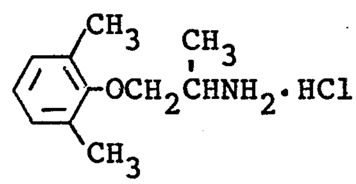

U. S. Patents Nos. 3,900,481 and 4,005,209 disclose "Flecanide" represented by the following formula.

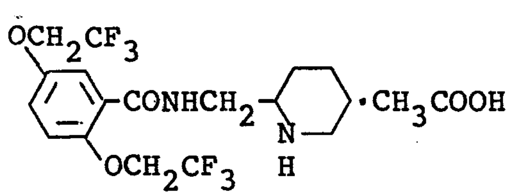

These patent documents, however, do not at all describe the compounds represented by formula (I) given hereinabove. Furthermore, the compounds described in the

prior literature tend to show undesirable actions including their side effects on the central nervous system.

The present inventors have made investigations in order to develop aminoalkyl-substituted benzene compounds having improved pharmacological activity, and succeeded in synthesizing the novel compounds of formula (I) not described in the literature. They have found that the novel compounds of formula (I) exhibit an excellent pharmacological efficacy for the treatment of diseases of the circulatory system, particularly arrhythmia, with markedly reduced side-effects on the central nervous system. It has also been found that the novel compounds of this invention show different pharmacological actions from conventional analogous compounds, particularly vasoactive pharmacological activity, are useful for the treatment of arrhythmia, have low toxicity, and can be used over an extended period of time.

It is an object of this invention therefore to provide the novel compounds of formula (I).

Another object of this invention is to provide a process for producing the compounds of formula (I), and a pharmaceutical composition comprising the compound of formula (I) as an active ingredient.

The above and other objects and advantages of this invention will become apparent from the following description.

In the novel compounds of this invention represented by formula (I)

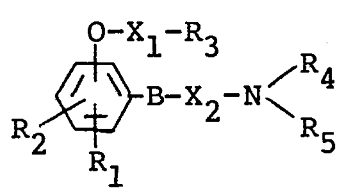

.....(I)

$R_1$, $R_2$, $R_3$, $R_4$, $R_5$, $X_1$, $X_2$ and B are as defined hereinabove. Preferred examples of $R_1$ to $R_5$ are shown below.

$R_1$ is a member selected from the class consisting of a hydrogen atom, $C_1$-$C_4$ alkyl groups, $C_1$-$C_4$ alkoxy groups, $C_2$-$C_4$ aliphatic acyl groups, halogen atoms and a

trifluoroethoxy group.

$R_2$ is a hydrogen atom or a $C_1$-$C_4$ alkyl group.

$R_3$ is $-ONO_2$, $-CF_3$ or F.

$R_4$ and $R_5$, independently from each other, represent a member selected from the class consisting of a hydrogen atom, $C_1$-$C_4$ alkyl groups, halogen-substituted benzoyl groups, phenyl-substituted $C_2$-$C_4$ aliphatic acyl groups and a pyridine carbonyl group. When taken together, $R_4$ and $R_5$ may form a piperidino, piperazino or morpholino ring together with the nitrogen atom to which they are bonded. The above ring may be substituted by a lower alkyl group, particularly a $C_1$-$C_2$ alkyl group.

Examples of the $C_1$-$C_4$ alkyl groups, preferably $C_1$-$C_2$ alkyl groups, for $R_1$, $R_2$, $R_3$, $R_4$ and $R_5$ are methyl, ethyl, n- or iso-propyl, and n-, iso-, sec- or tert-butyl. Examples of $C_1$-$C_4$ alkoxy groups, preferably $C_1$-$C_2$ alkoxy groups, for $R_1$ are alkoxy groups having the above-exemplified alkyl groups. Examples of the $C_2$-$C_4$ aliphatic acyl groups for $R_1$ are acetyl, propionyl and butyryl groups. In the halogen-substituted benzoyl groups for $R_4$ and $R_5$, examples of the halogen are Cl, Br, I and F. Examples of the phenyl-substituted $C_2$-$C_4$ aliphatic acyl groups in $R_4$ and $R_5$ are the same aliphatic acyl groups as exemplified for $R_1$ substituted by a phenyl group.

The alkylene group has 1 to 8 carbon atoms, preferably 1 to 6 carbon atoms. Examples of the alkylene group for $X_1$ and $X_2$ are methylene, ethylene, trimethylene, tetramethylene, pentamethylene, hexamethylene, ethylidene, propylidene and isopropylidene.

The compounds of formula (I) may be in the form of acid addition salts. Examples of the salts include salts of inorganic acids such as hydrochloric acid, sulfuric acid and phosphoric acid and salts of organic acids such as oxalic acid, tartaric acid, maleic acid, citric acid, fumaric acid, acetic acid, propionic acid, methanesulfonic acid and toluenesulfonic acid.

Examples of the compounds of formula (I) and their acid addition salts are shown below.

2-Dimethylamino-2'-methoxy-5'-(2-nitroxyethoxy)-acetanilide and its acid addition salts,

3-amino-2'-methoxy-5'-(2-nitroxyethoxy)-butyranilide and its acid addition salts,

3-amino-2'-methoxy-5'-(2,2,2-trifluoroethoxy)-butyranilide and its acid addition salts,

1-[2-methoxy-5-(2-nitroxyethoxy)phenoxy]-2-propanamine and its acid addition salts,

1-[2-methoxy-5-(2-nitroxyethoxy)phenoxy]-3-butanamine and its acid addition salts,

2-amino-2',6'-dimethyl-4'-(2-nitroxyethoxy)-propionanilide and its acid addition salts,

2-amino-2'-methoxy-5'-(2-nitroxypropoxy)-propionanilide and its acid addition salts,

2-(2-pyridinecarbonylamino)-2'-methoxy-5'-(2-nitroxyethoxy)-acetanilide and its acid addition salts,

3-amino-5'-(2-fluoroethoxy)-2'-methoxybutyranilide and its acid addition salts,

2-amino-2',6'-dimethyl-4'-(6-nitroxyhexyloxy)-propionanilide and its acid addition salts,

3-morpholino-2'-methoxy-5'-(2-nitroxyethoxy)-propionanilide and its acid addition salts,

2-morpholino-2'-methoxy-5'-(2-nitroxyethoxy)-acetanilide and its acid addition salts,

3-piperidino-2'-methoxy-5'-(2-nitroxyethoxy)-propionanilide and its acid addition salts,

2'-methoxy-3-(4-methylpiperazino)-5'-(2-nitroxyethoxy)-propionanilide and its acid addition salts,

2-(dimethylamino)-2'-methoxy-4'-(2-nitroxyethoxy)-acetanilide and its acid addition salts,

2-amino-2'-methoxy-5'-(2-nitroxyethoxy)-acetanilide and its acid addition salts,

3-amino-2'-methoxy-5'-(2-nitroxyethoxy)-propionanilide and its acid addition salts,

6-amino-2'-methoxy-5'-(2-nitroxyethoxy)-hexananilide and its acid addition salts,

2-amino-2'-methoxy-5'-(2-nitroxyethoxy)-propionanilide and its acid addition salts,

2-amino-2'-methyl-5'-(2-nitroxyethoxy)-propionanilide and its acid addition salts,

2-amino-2'-methyl-6'-(2-nitroxyethoxy)-propionanilide and its acid addition salts,

3-amino-2'-methyl-5'-(2-nitroxyethoxy)-butyranilide and its acid addition salts,

2-amino-2'-methyl-5'-(3-nitroxypropoxy)-propionanilide and its acid addition salts,

2-amino-2'-fluoro-5'-(2-nitroxyethoxy)-propionanilide and its acid addition salts,

2-amino-2'-chloro-5'-(2-nitroxyethoxy)-propionanilide and its acid addition salts,

2-amino-2'-(2,2,2-trifluoroethoxy)-5'-(2-nitroxyethoxy)-propionanilide and its acid addition salts,

2-amino-2'-methyl-5'-(2-nitroxyethoxy)-butyranilide and its acid addition salts,

2-amino-3',5'-dimethyl-4'-(2-nitroxyethoxy)-propionanilide and its acid addition salts,

2-amino-2',6'-dimethyl-3'-(2-nitroxyethoxy)-propionanilide and its acid addition salts,

2-amino-2'-methyl-5'-(2-nitroxypropoxy)-propionanilide and its acid addition salts,

2-amino-2'-methyl-6'-(2-nitroxypropoxy)-propionanilide and its acid addition salts,

2-amino-2',6'-dimethyl-3'-(2-nitroxypropoxy)-propionanilide and its acid addition salts,

3-(2-chlorobenzoylamino)-2'-methoxy-5'-(2-nitroxyethoxy)-propionanilide and its acid addition salts,

6-(2-chlorobenzoylamino)-2'-methoxy-5'-(2-nitroxyethoxy)-hexananilide and its acid addition salts,

6-(3-phenylpropionylamino)-2'-methoxy-5'-(2-nitroxyethoxy)-hexananilide and its acid addition salts,

2-(2-chlorobenzoylamino)-2'-methoxy-5'-(2-nitroxyethoxy)-propionanilide and its acid addition salts,

2-amino-2',6'-dimethyl-4'-(2-fluoroethoxy)-propionanilide and its acid addition salts,

2-amino-2'-methyl-5'-(2,2,2-trifluoroethoxy)-propionanilide and its acid addition salts,

2-amino-2'-methyl-6'-(2,2,2-trifluoroethoxy)-propionanilide and its acid addition salts,

3-amino-2'-methyl-5'-(2,2,2-trifluoroethoxy)-butyranilide and its acid addition salts,

2-amino-2',6'-dimethyl-4'-(2,2,2-trifluoroethoxy)-propionanilide and its acid addition salts,

1-[2-methoxy-5-(2,2,2-trifluoroethoxy)phenoxy]-2-propanamine and its acid addition salts,

1-[2-methyl-5-(2-nitroxyethoxy)phenoxy]-2-propanamine and its acid addition salts,

1-[5-(nitroxyethoxy)-2-(2,2,2-trifluoroethoxy)-phenoxy]-2-propanamine and its acid addition salts,

2-(2-aminopropoxy)-4-(2-nitroxyethoxy)acetophenone and its acid addition salts,

1-[2,6-dimethyl-4-(2-nitroxyethoxy)phenoxy]-2-propanamine and its acid addition salts,

1-[2-methoxy-4-(2-nitroxyethoxy)phenoxy]-2-propanamine and its acid addition salts,

1-[2-fluoro-4-(2-nitroxyethoxy)phenoxy]-2-propanamine and its acid addition salts,

1-[2-methoxy-5-(2-nitroxyethoxy)pohenoxy]-2-butanamine and its acid addition salts,

1-[2-methoxy-5-(2-nitroxyethoxy)phenoxy]-2-piperidinepropane and its acid addition salts,

1-[3-fluoro-4-(2,2,2-trifluoroethoxy)phenoxy]-2-propanamine and its acid addition salts,

1-[2-methoxy-3,5-bis(2,2,2-trifluoroethoxy)-phenoxy]-2-propanamine and its acid addition salts, and

2-amino-2',6'-dimethyl-4'-(3-nitroxypropoxy)-propionanilide and its acid addition salts.

The compounds of formula (I) in accordance with this invention can be produced by reacting a compound of the following formula (II)

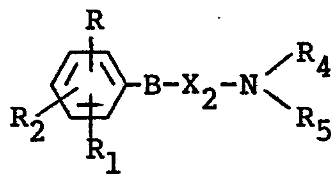

.....(II)

wherein $R_1$, $R_2$, $R_4$, $R_5$, $X_2$ and B are as defined with regard to formula (I), and R represents -OH, its alkali metal (e.g., Na or K) salt, or a halogen atom,

with a compound of the following formula (III)

$$R_3-X_1-Q \qquad .....(III)$$

wherein $R_3$ and $X_2$ are as defined above with regard to formula (I), and Q represents a halogen atom or a sulfonic acid ester residue (e.g., a tosyl, mesyl or benzenesulfonyl group) when R in formula (II) is -OH or its alkali metal salt, and -OH when R is a halogen atom.

The reaction can be carried out by contacting the compound of formula (II) with the compound of formula (III) in an inert organic solvent such as dimethylformamide. Preferably, the reaction is carried out in the presence of an alkali such as potassium carbonate or sodium hydroxide. The reaction temperature and time can be properly chosen, and for example temperatures ranging from room temperature to the boiling point of the solvent can be employed. The reaction time is, for example, about 30 minutes to about 6 hours. The amounts of the compound of formula (II) and the compound of formula (III) can be properly selected, and, for example, the compound of formula (III) may be used in an amount of about 1 to about 3 moles per mole of the compound of formula (II).

The compound of formula (II) used in the reaction can be produced by utilizing techniques known per se. For example, a compound of formula (II) in which B is -NHCO- (wherein the nitrogen atom of -NHCO is bonded to a carbon atom of the benzene ring) can be produced by reacting a substituted aniline of the following formula

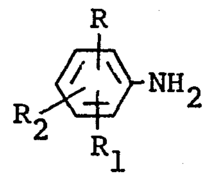

wherein $R_1$, $R_2$ and R are as defined above, with a halogeno-aliphatic carboxylic acid ($HOOC-X_2-Hal$ in which Hal represents a halogen atom such as Cl or Br and $X_2$ is as defined hereinabove) or an amino aliphatic carboxylic acid of the following formula

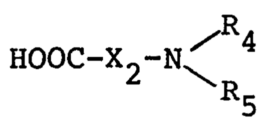

wherein $R_4$, $R_5$ and $X_2$ are as defined above, provided that when both $R_4$ and $R_5$ are hydrogen atoms, it is preferred to protect one of them by a protecting group such as a benzyloxycarbonyl group which is known as a protective group for the amino group, or a reactive derivative thereof, and when the halogeno aliphatic carboxylic acid is used, further reacting the product with a compound of the formula

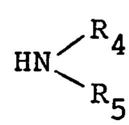

wherein $R_4$ and $R_5$ are as defined above.

Examples of the reactive derivative are acid anhydrides or acid halides of the halogeno aliphatic carboxylic acid or the amino aliphatic carboxylic acid. The reaction of the substituted aniline with the carboxylic acid may be carried out preferably in the presence of a dehydrating agent such as dicyclohexyl carbodiimide.

The reaction of the substituted aniline with the carboxylic acid or its reactive derivative may be carried out in a suitable organic solvent at a temperature of from room temperature to the boiling point of the solvent used. Examples of the solvent are tetrahydrofuran and methylene chloride. The reaction time can be properly chosen, and is, for example, about 30 minutes to about 6 hours. When the amino aliphatic carboxyic acid having the protective group is used, the reaction product may be hydrolyzed by a method known per se to eliminate the protective group.

The further reaction with the compound of the formula

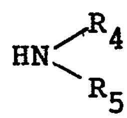

in the case of using the halogeno aliphatic carboxyic acid may be carried out, for example, by contacting the reaction product with the above compound in an inert organic solvent such as ethanol. The reaction temperature can be properly chosen, and may, for example, be room temperature to about $70^{o}C$. The reaction time may, for example, be about 10 minutes to about 2 hours.

A compound of formula (II) in which B is -O- can also be produced by utilizing a method known per se. For example, it can be produced by reacting a substituted phenol represented by the following formula

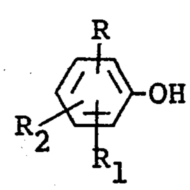

wherein $R_1$, $R_2$ and R are as defined above, with a sulfonic acid ester of an amino aliphatic alcohol represented by the following formula

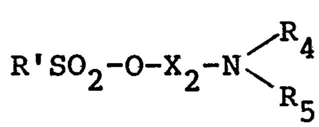

wherein $R_4$, $R_5$ and $X_2$ are as defined hereinabove, provided that when $R_4$ and $R_5$ are both hydrogen atoms, one of them is preferably protected by a protecting group such as a benzyloxycarbonyl group which is known as an amino protective group,

and when the product has the protective group, hydrolyzing it by a method known <u>per se</u> to eliminate it.

This reaction can be carried out by contacting the two reactants in a suitable inert organic solvent such as dimethylformamide. The reaction temperature and time can be selected properly. For example, temperatures from room temperature to the boiling point of the solvent and time periods ranging from about 30 minutes to about 6 hours may be employed.

The compound of formula (II) in which B is -O- can also be produced by reacting the aforesaid substituted phenol with a halogenoalkylketone such as a halogenoacetone or a halogenomethyl ethyl ketone, reducing the keto group to a hydroxyl group, thereafter treating the product with a sulfonic acid such as methanesulfonic acid or toluenesulfonic acid to convert the hydroxyl group to a sulfonate group, and converting the sulfonate group to an amino group by reacting the product with ammonia, for example, in a sealed tube at about 80°C for about 6 hours. The reaction of the substituted phenol and the halogenoalkylketone can be carried out by contacting both in an inert organic solvent such as acetone in the presence of an alkali such as potassium carbonate and potassiuum iodide, for example under heat refluxing for about 1 to about 4 hours. The reduction of the keto group of the resulting product can be effected by using a reducing agent such as sodium borohydride. Preferably, the conversion to the sulfonate group is carried out at low temperatures, for example by contacting with a sulfonic acid under ice cooling.

A compound of formula (I) in accordance with this invention in which both $R_4$ and $R_5$ are hydrogen atoms may

be converted bo a compound of formula (I) in which $R_4$ and/or $R_5$ is a lower alkyl group or a halogen-substituted benzoyl group by alkylating or acylating it in a customary manner.

A compound of formula (I) in which $R_3$ is nitroxy ($-ONO_2$) can also be produced by using a compound of formula (III) in which $R_3$ is a hydroxyl group, and converting the resulting compound of formula (I) in which $R_3$ is a hydroxyl group into a nitrate ester in a customary manner.

The compound of formula (I) so produced can be converted to its acid addition salt by contacting it with an inorganic or organic acid. The reaction may be carried out by contacting the compound of formula (I) with an inorganic or organic acid in the presence or absence of a solvent. The reaction temperature and time can be properly selected. For example, temperatures from room temperatures to about 50$^{o}$C and periods of 1 to 24 hours may be employed. Examples of the inorganic or organic acids may be those already exemplified hereinabove with regard to the acid addition salts.

According to this invention there can be provided a pharmaceutical composition comprising an antiarrhythmically effective amount of the aminoalkyl-substituted benzene derivative of formula (I) or its pharmaceutically acceptable acid addition salt and a pharmaceutically acceptable diluent or carrier.

The compounds of formula (I) and the pharmaceutically acceptable acid addition salts thereof have reduced side-effects on the central nervous system as compared with conventional analogous compounds and exhibit vasoactive pharmacological activity which the conventional compounds do not. For this reason, they are useful for treating arrhythmia and can be used for an extended period of time. Furthermore, they have low toxicity.

The compounds of formula (I) in accordance with this invention can be administered orally or by injection as

antiarrhythmic drugs. Their activity is long-lasting and are useful for the treatment and prevention of arrhythmia.

Preferably, the compound of formula (I) or its acid addition salt is used as an antiarryhythmic drug in the form of tablets, sublingual tablets, capsules, powders, granules, suppositories, liquids, injecting preparations, suspensions, etc. prepared by using pharmaceutically acceptable diluents or carriers such as vehicles, binders, solvents and emulsifiers.

Examples of the vehicles are starches such as potato starch, wheat starch and corn starch, sugars such as lactose, sucrose, glucose, mannitol and sorbitol, celluloses such as crystalline cellulose, carboxy methyl cellulose calcium and hydroxypropyl cellulose, and inorganic substances such as calcium phosphate, calcium sulfate, calcium carbonate and talc.

Examples of the binders are starch, gelatin, gum arabic, methyl cellulose, carboxy methyl cellulose sodium, polyvinyl pyrrolidone and hydroxypropyl cellulose.

Examples of the emulsifiers are polyhydric alcohol ester-type nonionic surface-active agents and polyoxyethyene-type noninonic surface-active agents, such as fatty cid monoglycerides, sorbitan fatty acid esters, sucrose and polyglycerol fatty acid esters.

The dosage of the pharmaceutical composition of this invention varies depending upon the condition of a patient. Usually, it is 10 to 1,000 mg, preferably 100 to 500 mg, per day as the compound of formula (I) or its pharmaceutically acceptable acid addition salt in the case of oral administration.

The acute toxicity of the compounds of formula (I) or the pharmaceutically acceptable acid addition salts thereof in accordance with this invention corresponds to an $LD_{50}$ value of 500 to 1,000 mg/kg in oral administration to mice, and therefore, they have very low toxicity.

The pharmacological activity of the compounds of

this invention was tested, and the results are shown below.

Test Example 1

Activity on Chloroform-Induced Arrhythmia:-

ICR-strain mice having a body weight of 20 to 30 g (10 per group) were used, and the test compound was administered orally in a dose of 100 mg/kg. The mice were put in a 1-liter beaker, and observed for 20 minutes for their symptoms or conditions. Then, they were placed in a vessel saturated with a vapor of chloroform, and the time which elapsed until the animals ceased to breathe was measured. After the stopping of the breathing, the mice were taken out from the vessel and their cardiograms were taken. The rates of onset of ventricular tachycardia (VT) and ventricular fibrillation (VF) and their conditions were observed. The duration of activity was determined by using as a standard the time during which the mice were put in the chloroform-saturated vessel. The conditions were indicated in Table 1 below when the same tendency was observed in all the subjects tested. Lidocaine was used as a comparative drug. The results are shown in Table 1.

Table 1

| Drug No.(*) | Antiarrhythmic activity | | | Condition |
|---|---|---|---|---|
| | Rate of onset of VT | Rate of onset of VF | Duration of activity (**) | |
| 3 | 2/10 | 0/10 | +++ | Sedation, ataxia |
| 4 | 4/10 | 0/10 | ++ | |
| 6 | 1/10 | 0/10 | ++ | Weak sedation |
| 22 | 0/10 | 0/10 | ++ | Sedation |
| 24 | 2/10 | 0/10 | + | Weak sedation |
| 38 | 5/10 | 2/10 | + | Weak sedation |
| 40 | 0/10 | 0/10 | +++ | |
| 41 | 0/10 | 1/10 | +++ | Sedation |
| Lidocaine | 0/10 | 2/10 | + | Weak sedation |

(*): The numbers are those of Examples given hereinbelow.

(**): The duration of activity is shown by the relative intensity to the duration time of Lidocaine which is represented by +.

Test Example 2

Hypotensive action:-

Mice having a body weight of about 30 g (5 per group) were used. Under anaesthesia with pentobarbital, the carotid was removed from each animal. A cannula was inserted into the animal and connected to a pressure transducer. A tracheal cannula was applied, and the animal was fixed on its back. After the animals awoke from anesthesia, their average blood pressures were measured by the pressure transducer.

The drug was orally administered in a dose of 30 mg/kg, and changes in the blood pressure were measured. Physiological saline was administered to a control group. The results are shown in Table 2.

Table 2

Average blood pressure (mmHg)

| | Before administration | Time which elapsed after the administration (minutes) | | | |
|---|---|---|---|---|---|
| | | 10 | 30 | 60 | 120 |
| Drug group | 86.3 ±6.8 | 73.3 ±8.5 (*) | 76.4 ±7.8 (*) | 77.4 ±8.1 (*) | 80.3 ±8.0 (**) |
| Control group | 95.9 ±5.1 | 93.0 ±4.5 | 93.9 ±4.2 | 94.8 ±4.9 | 93.9 ±4.4 |

(*): $p<0.01$; (**): $p<0.05$

The drug used in Test Example 2 was the compound prepared in Example 6.

Example 1

2-Dimethylamino-2'-methoxy-5'-(2-nitroxyethoxy)-acetanilide:-

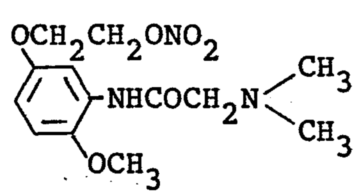

(a) In 80 ml of anhydrous tetrahydrofuran were dissolved 4.0 g of 5-ethoxycarbonyloxy-2-methoxyaniline and 2.1 g of triethylamine, and 20 ml fo an anhydrous tetrahydrofuran solution of 4.16 g of bromoacetyl bromide was added. The mixture was stirred at room temperature for 30 minutes. After the reaction, the insoluble materials were removed by filtration, and the solvent was evaporated to

give 6.9 g of a crude product. The crude product was dissolved in 14 g of a 40% aqueous solution of dimethylamine and 45 ml of ethanol, and the solution was stirred at room temperature for 30 minutes. After the reaction, the solvent was evaporated under reduced pressure. The residue was dissolved in ethyl acetate, washed with water, and dried. The solvent was evaporated. Recrystallization of the residue from acetone/n-hexane gave 3.0 g (yield 71.4%) of 2-dimethylamino-5'-hydroxy-2'-methoxyacetanilide as pale brown prisms having a melting point of 189 to 191°C.

(b) To 733 mg of the resulting compound were added 405 mg of ethylene carbonate, 1 ml of tetraethyl ammonium iodide and 1 ml of dimethylformamide, and the mixture was stirred at 145°C for 2 hours. After the reaction, the reaction mixture was dissolved in ethyl acetate, washed with water and dried. The solvent was evaporated, and the residue was purified by silica gel column chromatography (eluent: chloroform/methanol=40/1). The purified product was converted to a maleate salt in a customary manner and recrystallized from a mixture of acetone and ether to give 433 mg (yield 53.5%) of 2-dimethylamino-5'-(2-hydroxyethoxy)-2'-methoxyacetanilide maleate as a pale brown powder having a melting point of 107 to 108°C.

(c) The resulting hydroxyethoxy compound (1.0 g) was dissolved in 25 ml of anhydrous pyridine, and 1.86 g of tosyl chloride was added. The mixture was stirred at room temperature for 75 minutes. Then, water was added, and the mixture was further stirred. After the reaction, pyridine was evaporated under reduced pressure, and an aqueous solution of sodium bicarbonate was added to the residue. The mixture was extracted with ethyl acetate. The extract was washed with an aqueous solution of sodium chloride and dried. Ethyl acetate was evaporated to give 1.3 g (yield 82.8%) of 2-dimethylamino-2'-methoxy-5'-(2-tosyloxyethoxy)-acetanilide.

To 1.5 g of the resulting tosyloxyethoxy compound

were added 6.4 g of sodium iodide and 43 ml of dimethylformamide. The mixture was stirred at 60°C for 1 hour. After the reaction, the reaction mixture was poured into water and extracted with ethyl acetate. The extract was washed with an aqueous solution of sodium chloride, and dried. The solvent was evaporated to give 1.38 g of 2-dimethylamino-5'-(2-iodoethoxy)-2'-methoxyacetanilide.

(d) To 1.38 g of the resulting iodoethoxy compound were added 4.2 g of silver nitrate and 20 ml of acetonitrile. The solution was stirred at 60°C for 15 minutes. The solvent was evaporated, and water and ethyl acetate were added. The mixture was filtered on Celite, and the ethyl acetate layer was separated. The extract was dried, and the solvent was evaporated. The residue was purified by silica gel column chromatography (eluent: chloroform/methanol=70/1) and recrystallized from a mixture of ether and n-hexane to give 354 mg (yield 63.6 %) of the desired compound as pale brown prisms having a melting point of 69 to 72°C.

IR: $\nu_{max}^{KBr}$ $cm^{-1}$

1640 ($ONO_2$), 1690 (NHCO)

NMR: $\delta$ ($CDCl_3$)

2.38 (6H, s, 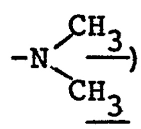)

3.08 (2H, s, $-COCH_2-$)

3.85 (3H, s, $-OCH_3$)

4.20 (2H, t, J=4Hz, $-OCH_2-$)

4.76 (2H, t, J=4Hz, $-CH_2ONO_2$)

6.50 (1H, d-d, J=3Hz, 8Hz, H of the aromatic ring)

6.76 (1H, d, J=8Hz, H of the aromatic ring)

8.10 (1H, d, J=3Hz, H of the aromatic ring)

Example 2

3-Amino-2'-methoxy-5'-(2-nitroxyethoxy)-butyranilide maleate:-

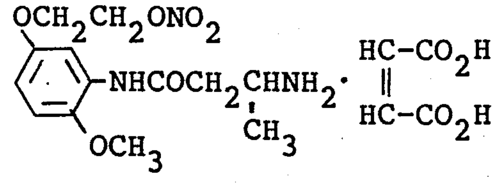

(a) To 300 ml of methylene chloride were added 13.5 g of 5-ethoxycarbonyl-2-methoxyaniline, 16.62 g of 3-t-butoxycarbonylamino-n-butyric acid and 16.9 g of N,N'-dicyclohexyl carbodiimide, and the mixture was stirred at room temperature for 3 hours. The insoluble materials were removed by filtration, and the solvent was evaporated. The resulting reddish brown oily product was dissolved in 210 ml of methanol and 71 ml of 1N sodium hydroxide was added. The mixture was stirred at room temperature for 20 minutes. The reaction mixture was neutralized with acetic acid, and the solvent was evaporated. The residue was dissolved in ethyl acetate and successively washed with an aqueous solution of sodium bicarbonate and water. The product was then dried and concentrated to give 18.52 g (yield 89.3%) of 3-(t-butoxycarbonylamino)-5'-hydroxy-2'-methoxybutyranilide as colorless crystals having a melting point of 179 to 181°C.

(b) The above product was worked up in the same way as in Example 1, (a) to (d) to give 3-(t-butoxycarbonylamino)-2'-methoxy-5'-(2-nitroxyethoxy)-butyranilide as colorless crystals having a melting point of 122 to 123°C.

(c) Thirty milliliters of 6N-HCl was added to 180 ml of a tetrahydrofuran solution of 7.5 g of the resulting compound, and the solution was stirred at 70°C for 1 hour. After the reaction, the reaction mixture was neutralized with 1N sodium hydroxide. The solvent was evaporated. 1N-sodium hydroxide was further added to adjust the pH of the residue to 9, and it was then extracted with ethyl acetate. The extract was separated and dried. The solvent was evaporated to give an oily product. The oily product was purified by silica gel column chromatography (eluent:

chloroform/methanol=30/1-2/1,containing ammonia) to give 3.62 g (yield 63.7%) of a brown viscous oily product. This compound was converted to a maleate salt in a customary manner, and recrystallized from a mixture of ethyl acetate and ether to give 5.79 g (yield 94.1%) of the desired compound as a pale yellow powder.

IR: $\nu_{max}^{KBr}$ $cm^{-1}$:
630 ($ONO_2$), 1690 (NHCO)

NMR: $\delta$ ($CD_3OD$)
1.39 (3H, d, J=7Hz, $-CH_3$)
2.72 - 2.88 (2H, m. $-COCH_2-$)
3.48 - 3.92 (4H, m, -CONH-, $-OCH_3$)
4.16 - 4.30 (2H, m, $-OCH_2-$)
4.56 - 5.10 (7H, m, $-CH_2ONO_2$, -NHCO, $-NH_2$, 2 COOH)
6.22 (2H, s, -CH=CH-)
6.68 (1H, d-d, J=2.5Hz, 8Hz, H of the aromatic ring)
6.92 (1H, d, J=8Hz, H of the aromatic ring)
7.77 (1H, d, J=2.5Hz, H of the aromatic ring)

Example 3

3-Amino-2'-methoxy-5'-(2,2,2-trifluoroethoxy)-butyranilide·hemifumarate:-

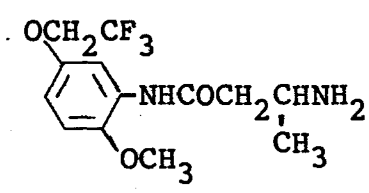

An anhydrous dimethylformamide solution (12 ml) of 3.98 g of 3-(t-butoxycarbonylamino)-5'-hydroxy-2'-methoxybutylanilide, 2,2,2-trifluoroethyltrifluoromethane sulfonate (1.5 equivalent moles) and 2.5 g of potassium carbonate was stirred at 50°C for 1 hour. After the reaction, ethyl acetate was added, followed by washing with water and drying. The solvent was evaporated, and

the residue was purified by silica gel column chromatography (eluent:chloro form/methanol=100/1) to give 4.02 g (yield 80.6%) of 3-(t-butoxycarbonylamino)-2'-methoxy-5'-(2,2,2-trifluoroethoxy)-butyranilide as pale brown crystals having a melting point of 131 to 132.5°C.

The product was worked up in the same way as in Example 2 to deprotect it. When 1.741 g of fumaric acid was added to an ethanol solution of 4.60 g of a free base of the title compound, there was obtained 5.25 g (yield 96.0%) of the title compound as a colorless powder having a melting point of 179 to 180°C.

IR: $\nu_{max}^{KBr}$ $cm^{-1}$:

1670 (NHCO), 3300 ($NH_2$)

NMR: $\delta$ ($CD_3OD$)

1.38 (3H, d, J=6Hz, $-CH_3$)

2.78 (2H, d, J=6Hz, $-COCH_2-$)

3.70 (1H, q, J=6Hz, -CHN<)

3.84 (3H, s, $-OCH_3$)

4.41 (2H, q, J=8Hz, $-OCH_2-$)

4.87 (5H, s, -NHCO-, $-NH_2$, 2(-COOH))

6.63 (1H, s, -CH<)

6.72 (1H, d-d, J=2.5Hz. 8Hz. H of the aromatic ring)

6.92 (1H, d, J=8Hz, H of the aromatic ring)

7.82 (1H, d, J=2.5Hz, H of the aromatic ring)

Example 4

1-[2-Methoxy-5-(2-nitroxyethoxy)phenoxy]-2-propanamine·maleate:-

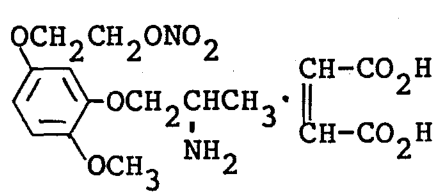

(a) 50.4 g of 5-(2-acetoxyethoxy)-2-methoxyacetophenone obtained by the action of acetyl chloride on 2-(p-methoxyphenoxy)ethyl acetate was added to 200 ml of

a methanol solution of 12.3 g of potassium hydroxide. The mixture was stirred at room temperature for 30 minutes. After the reaction, the solvent was evaporated, and water was added to the resulting residue. The mixture was extracted with ethyl acetate. The extract was washed successively with dilute hydrochloric acid and an aqueous solution of sodium chloride, and dried. The solvent was evaporated to give 37.6 g (yield 89.5%) of 5-(2-hydroxyethoxy)-2-methoxyacetophenone.

An acetonitrile solution (300 ml) of 21 g of the resulting compound was cooled to -25°C, and with stirring, acetyl nitrate was gradually added dropwise. After the reaction, the reaction mixture was poured into an iced aqueous solution of sodium bicarbonate saturated with sodium chloride, and the organic layer was separated. The aqueous layer was extracted with ethyl acetate, and the extract was combined with the organic layer. The mixture was successively washed with a saturated aqueous solution of sodium bicarbonate and a saturated aqueous solution of sodium chloride, and dried. The solvent was evaporated to give brown crystals. The crystals were purified by using silica gel column chromatography (eluent: benzene/acetone=9/1) to give 18 g (yield 70%) of 2-methoxy-5-(2-nitroxyethoxy)-acetophenone as colorless needles having a melting point of 58.5 to 59.5°C.

(b) The resulting nitroxyethoxy compound (15.3 g) was added to 50 g of 40% peracetic acid with stirring and ice cooling. The mixture was stirred for 1 hour and then at room temperature for 3 hours. After the reaction, the reaction mixture was poured into ice water, and extracted with chloroform. The extract was successively washed with a dilute aqueous solution of sodium hyposulfite, a saturated aqueous solution of sodium bicarbonate and a saturated aqueous solution of sodium chloride, and dried. The solvent was evaporated. The residue was dissolved in 50 ml of methylene chloride, and 10 ml of pyrrolidine was added.

The mixture was stirred at room temperature for 10 minutes. After the reaction, the reaction mixture was extracted twice with 50 ml of 1N sodium hydroxide. Under ice cooling, 2N hydrochloric acid was added to acidify the extracts, followed by extraction with chloroform. The chloroform layer was washed successively with a saturated aqueous solution of sodium bicarbonate and a saturated aqueous solution of sodium chloride, and dried. The solvent was evaporated, and the residue was purified by silica gel column chromatography (eluent: benzene-acetone=9/1) to give 11.1 g (yield 81.4%) of 2-methoxy-5-(2-nitroxyethoxy)-phenol as colorless crystals having a melting point of 63.5 to 64.5°C.

(c) One hundred milliliters of an acetone solution of 8.01 g of the resulting phenol compound, 5.8 g of potassium carbonate, 1.05 g of potassium iodide and 5.8 g of monochloroacetone was stirred for 3 hours under heat refluxing. The reaction mixture was filtered, and the solvent was evaporated from the resulting mother liquor. Water was added to the residue, and the mixture was extracted with ethyl acetate. The extract was washed with water, and dried. The solvent was evaporated. The resulting crystals were purified by silica gel column chromatography (eluent: benzene-acetone=9:1), and recrystallized from benzene/n-hexane to give 8.6 g (yield 86.3%) of 1-[2-methoxy-5-(2-nitroxyethoxy)phenoxy]-2-propanone as colorless needles having a melting point of 76 to 77°C.

When 7.13 g of the compound was reduced with sodium borohydride in a customary manner, 7.1 g (yield 98%) of 1-[2-methoxy-5-(2-nitroxyethoxy)phenoxy]-2-propanol was obtained as a pale yellow oil.

(d) To 7.2 g of the resulting propanol compound were added 0.61 g of 4-dimethylaminopyridine and 50 ml of methylene chloride to dissolve it. With ice cooling and stirring, 3.03 g of triethylamine and 3.44 g of mesyl chloride were added dropwise, and the mixture was stirred for 1 hour. The reaction mixture was successively washed

with water, dilute hydrochloric acid, an aqueous solution of sodium bicarbonate and a dilute aqueous solution of sodium chloride, and dried. The solvent was evaporatd, and the resulting crystals were recrystallized from ethyl acetate/ n-hexane to give 8.3 g (yield 90.6%) of the mesyl ester as colorless crystals having a melting point of 97 to 98°C.

One gram of the mesyl ester was dissolved in 30 ml of ammonia-saturated methanol (15% W/V), and the solution was heated at 80°C for 6 hours in a sealed tube. After the reaction, the solvent was evaporated. Chloroform was added to the residue, and the insoluble materials were removed by filtration. The mother liquor was extracted with 25 ml of 1N hydrochloric acid and then with 10 ml of 1N hydrochloric acid. The extracts were combined, washed with chloroform, adjusted to pH about 10 with potassium carbonate, and extracted with ethyl acetae. The extract was dried, and the solvent was evaporated. The resulting jelly-like residue was purified by silica gel thin-layer chromatography (developing solvent: chloroform/methanol= 95/5, containing ammonia) to give 74 mg (yield 22.2%) of a pale yellow oil. The product was converted to a maleate salt in a customary manner, and recrystallized from methylene chloride/ether to give the title compound as colorless crystals having a melting point of 56 to 60°C.

IR: $\nu_{max}^{film}$ $cm^{-1}$:
1630 ($ONO_2$), 3400 ($NH_2$)

NMR: $\delta$($CD_3OD$)
1.42 (3H, d, J=6Hz, $-C\underline{H_3}$)
3.72 (1H, m, $-C\underline{H}-$)
3.82 (3H, s, $-OC\underline{H_3}$)
4.08 (2H, m, $-OC\underline{H_2}-$)
4.20 (2H, m, $-OC\underline{H_2}CH_2-$)
4.80 (2H, m, $-CH_2ONO_2$)
6.20 (2H, s, $-C\underline{H}=C\underline{H}-$)
6.48 (1H, d-d, J=2Hz, 8Hz, H of the aromatic ring)

6.60 (1H, d, J=2Hz, H of the aromatic ring)

6.84 (1H, d, J=8Hz, H of the aromatic ring)

Mass: m/e

286 ($M^+$)

Example 5

1-[2-Methoxy-5-(2-nitroxyethoxy)phenoxy]-3-butanamine:-

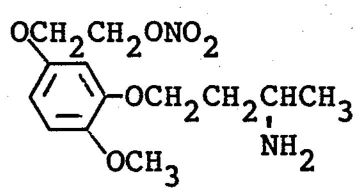

(a) 1.11 g of isovanilin was dissolved in 8.76 ml of a methanol solution of 1N-sodium methylate, and the solution was evaporated to dryness under reduced pressure. The dried product was dissolved in 10 ml of dimethylformamide. Then, 2 ml of a dimethylformamide solution of 2.54 g of 3-(t-butoxycarbonylamino)-butyl-1-methanesulfonate was added. The solution was stirred at 80°C for 1 hour. After cooling, the reaction mixture was poured into an aqueous solution of sodium chloride and extracted with ethyl acetate. The extract was washed with an aqueous solution of sodium chloride and dried. Then, the solvent was evaporated, and the residue was purified by silica gel column chromatography (eluent: chloroform/methanol=100/1). The resulting compound was dissolved in chloroform, washed successively with 10% sodium hydroxide and an aqueous solution of sodium chloride, and dried. The solvent was evaporated to leave 1.84 g (78.0%) of 3-[3-(t-butoxycarbonylamino)-butoxy]-4-methoxybenzaldehyde.

(b) To 1.76 g of the resulting aldehyde compound were added 1.28 g of m-chloroperbenzoic acid, 0.5 g of sodium bicarbonate and 30 ml of methylene chloride, and the mixture was stirred at room temperature for 1.5 hours. After the reaction, the reaction mixture was washed with an aqueous solution of sodium chloride. The solvent was evaporated,

and ammonia-methanol (15% W/V) was added to the residue. After standing for 5 minutes, the mixture was dried under pressure. Ethyl acetate was added to the residue, and the solution was washed with an aqueous solution of soludium chloride and then dried. The solvent was evaporated to give 1.51 g (yield 92.6%) of 3-[3-(t-butoxycarbonylamino)-butoxy]-4-methoxyphenol as an oil.

(c) The product was then worked up in the same way as in Example 1, (b) to (d) to hydroxyalkylate and nitroxylate it. The desired compound was obtained as an oil (yield 29%).

IR: $\nu_{max}^{film}$ $cm^{-1}$:

1620 ($ONO_2$)

MAss: m/e

300 ($M^+$)

NMR: $\delta$ ($CDCl_3$)

1.18 (3H, d, J=6Hz, $-CH_3$)

1.80 - 2.00 (2H, m, $-CH_2-$)

3.10 - 3.35 (1H, m, $-CH<$)

3.80 (3H, s, $-OCH_3$)

4.63 - 4.90 (2H, m, $-CH_2ONO_2$)

6.33 - 6.88 (3H, m, H of the aromatic ring)

The following compounds can be produced in the same manner as in Examples 1 to 5.

Example 6

2-Amino-2',6'-dimethyl-4'-(2-nitroxyethoxy)-propionanilide·hydrochloride

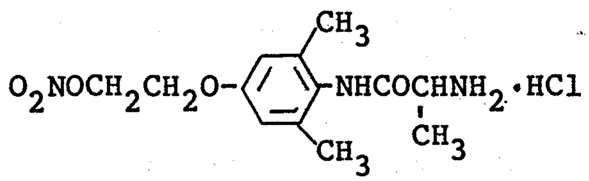

m.p.: 198.5-200.5°C, colorless needle-like crystals

NMR: δ ($CD_3OD$)

1.67 (3H, d, J=6Hz, $-CH_3$)
2.17 (6H, s, $-CH_3$)
4.07-4.32 (3H, m, -CH, $-OCH_2$)
4.70-4.90 (6H, m, $-CH_2ONO_2$, -NHCO-, $-NH_2$-, HCl)
6.67 (2H, s, H of the aromatic ring)

IR: $\nu_{max}^{KBr}$ $cm^{-1}$

1630 ($ONO_2$), 1670 (NHCO)

Example 7

2-Amino-2'-methoxy-5'-(2-nitroxypropoxy)-propionanilide

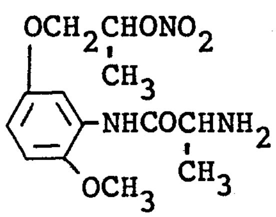

Yellowish brown oily product

NMR: δ ($CDCl_3$)

1.40 (3H, d, J=6Hz, $-CH_3$)
1.43 (3H, d, J=6Hz, $-CH_3$)
3.82 (3H, s, $-OCH_3$)
3.63 (1H, q, J=6Hz, -COCH-)
4.05 (2H, d, J=6Hz, $-OCH_2$-)
5.25-5.60 (2H, m, $-CH_2ONO_2$)

IR: $\nu_{max}^{film}$ $cm^{-1}$

1620 ($ONO_2$), 1680 (NHCO)

Example 8

2-(2-Pyridine carbonylamino)-2'-methoxy-5'-(2-nitroxyethoxy)acetoanilide

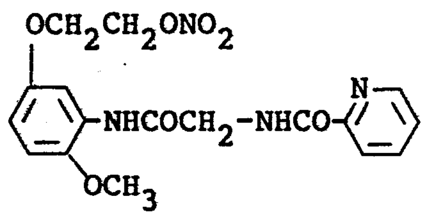

m.p. 172-173$^{\circ}$C, yellowish needle-like crystals

NMR: $\delta$ (DMSO-$d_6$)

3.75 (3H, s, $-OCH_3$)

4.06-4.32 (4H, m, $-COCH_2-$, $-OCH_2-$)

4.76-4.96 (2H, m, $-CH_2ONO_2$)

6.60 (1H, d-d, J=3Hz, 9Hz, H of the aromatic ring)

6.92 (1H, d, J=9Hz, H of the aromatic ring)

7.50-7.70 (1H, m, H of the aromatic ring)

7.76 (1H, d, J=3Hz, H of the aromatic ring)

7.86-8.17 (2H, m, H of the aromatic ring)

8.64 (1H, d, J=4Hz, H of the aromatic ring)

8.90-9.32 (2H, m, -NHCO-)

IR: $\nu_{max}^{KBr}$ $cm^{-1}$

1640 ($ONO_2$), 1700 (NHCO)

Example 9

3-Amino-5'-(2-fluoroethoxy)-2'-methoxybutyranilide

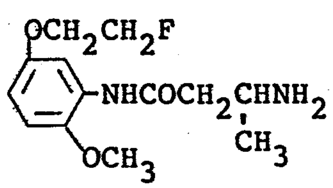

Pale yellow viscous oily product

NMR: $\delta$ ($CDCl_3$)

1.19 (3H, d, J=6Hz, $-CH_3$)

1.70 (2H, br, s, $-NH_2$)

2.29 (1H, d-d, J=8Hz, 16Hz, -COCH< )

2.51 (1H, d-d, J=4Hz, 16Hz, -COCH< )

3.26-3.68 (1H, m, -CH-)

3.81 (3H, s, $-OCH_3$)

4.00-4.13 (1H, m, -OCH-)

4.27-4.41 (1H, m, -OCH-)

4.41-4.57 (1H, m, -CHF)

4.89-5.04 (1H, m, -CHF)

6.59 (1H, d-d, J=3Hz, 8Hz, H of the aromatic ring)

6.79 (1H, d, J=8Hz, H of the aromatic ring)

8.14 (1H, d, J=3Hz, H of the aromatic ring)

9.35 (1H, m, -NHCO-)

IR: $\nu_{max}^{film}$ $cm^{-1}$

1665 (NHCO), 3350 ($NH_2$)

Example 10

2-Amino-2',6'-dimethyl-4'-(6-nitroxyhexyloxy)-propionanilide·hydrochloride

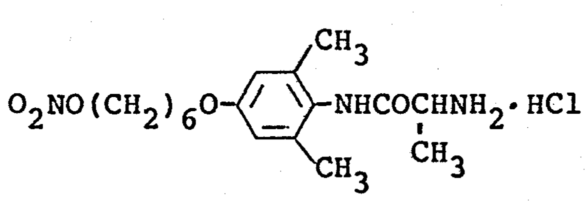

m.p. 168-170°C (methylene chloride-ether), colorless crystals)

IR: $\nu_{max}^{KBr}$ $cm^{-1}$

1615 ($ONO_2$), 1665 (NHCO)

Example 11

3-Morpholino-2'-methoxy-5'-(2-nitroxyethoxy)-propionanilide·hydrochloride

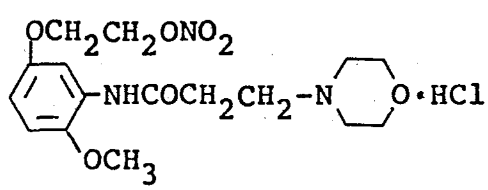

m.p. 168-169°C (ether-methanol), colorless fine needle-like crystals

NMR: $\delta$ ($CDCl_3+CD_3OD$)

3.84 (3H, s, $-OCH_3$)

4.80 (2H, t, J=4Hz, $-CH_2ONO_2$)

6.62 (1H, d-d, J=3Hz, 8Hz, H of the aromatic ring)

6.84 (1H, d, J=8Hz, H of the aromatic ring)

7.78 (1H, d, J=3Hz, H of the aromatic ring)

IR: $\nu_{max}^{KBr}$ $cm^{-1}$

1630 ($ONO_2$), 1640 (NHCO)

Mass: m/e

369 ($M^+$)

Example 12

2-Morpholino-2'-methoxy-5'-(2-nitroxyethoxy)-acetoanilide·hydrochloride

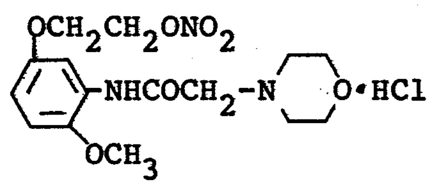

m.p. 160-161°C (decomposition)(ether-methanol), colorless fine needle-like crystals

NMR: δ ($CDCl_3$+$CD_3OD$)

3.30-3.70 (6H, m, $-\dot{N}CH_2-$)

3.84 (3H, s, $-OCH_3$)

4.80 (2H, t, J=6Hz, $-CH_2ONO_2$)

6.64 (1H, d-d, J=3Hz, 8Hz, H of the aromatic ring)

6.85 (1H, d, J=8Hz, H of the aromatic ring)

7.76 (1H, d, J=3Hz, H of the aromatic ring)

IR: $\nu_{max}^{KBr}$ $cm^{-1}$

1630 ($ONO_2$), 1650 (NHCO)

Example 13

3-Piperidino-2'-methoxy-5'-(2-nitroxyethoxy)-propionanilide·hydrochloride

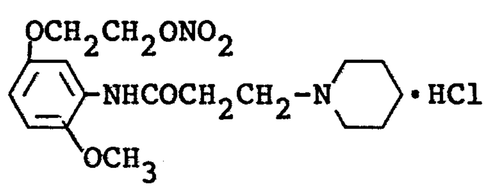

m.p. 163-164°C (ether-methanol), pale brown fine needle-like crystals

NMR: $\delta$ ($D_2O$)

1.40-2.30 (6H, m, $-C\underline{H_2}-$)

3.95 (3H, s, $-OC\underline{H_3}$)

4.25-4.47 (2H, m, $-OC\underline{H_2}-$)

4.80-5.00 (2H, m, $-C\underline{H_2}ONO_2$)

6.87 (1H, d-d, J=3Hz, 8Hz, H of the aromatic ring)

7.08 (1H, d, J=8Hz, H of the aromatic ring)

7.55 (1H, d, J=3Hz, H of the aromatic ring)

IR: $\nu_{max}^{KBr}$ $cm^{-1}$

1640 ($ONO_2$), 1690 (NHCO)

Example 14

2'-Methoxy-3-(4-methylpiperadino)-5'-(2-nitroxyethoxy)propionanilide·hydrochloride

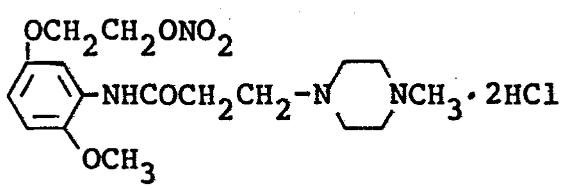

m.p. 147-151°C (decomposition)(ether-methanol), pale yellow powder

NMR: $\delta$ ($D_2O$)

3.12 (3H, s, $>NC\underline{H_3}$)

3.50-4.00 (10H, m, $>NC\underline{H_2}-$)

3.88 (3H, s, $-OC\underline{H_3}$)

4.83-5.00 (2H, m, $-C\underline{H_2}ONO_2$)

4.25-4.45 (2H, m $-OC\underline{H_2}-$)

6.85 (1H, d-d, J=3Hz, 8Hz, H of the aromatic ring)

7.05 (1H, d, J=8Hz, H of the aromatic ring)

7.45 (1H, d, J=3Hz, H of the aromatic ring)

IR: $\nu_{max}^{KBr}$ $cm^{-1}$

1630 ($ONO_2$), 1680 (NHCO)

Example 15

2-(Dimethylamino)-2'-methoxy-4'-(2-nitroxyethoxy)acetoanilide

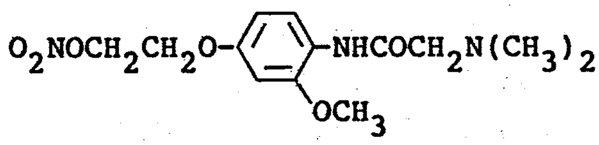

Brown viscous oily product

NMR: $\delta$ ($CDCl_3$)

2.36 (6H, s, $-N(CH_3)_2$)

3.05 (2H, s, $-COCH_2-$)

3.85 (3H, s, $-OCH_3$)

4.20 (2H, t, J=4Hz, $-OCH_2-$)

4.77 (2H, t, J=4Hz, $-CH_2ONO_2$)

6.30-8.35 (3H, m, H of the aromatic ring)

IR: $\nu_{max}^{film}$ $cm^{-1}$

1630 ($ONO_2$), 1680 (NHCO)

Example 16

2-Amino-2'-methoxy-5'-(2-nitroxyethoxy)-acetoanilide

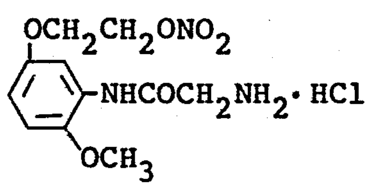

m.p. 167-168°C (ethanol), pale brown prism crystals

NMR: $\delta$ ($CD_3OD$)

3.84 (3H, s, $-OCH_3$)

3.92 (2H, s, $-COCH_2$)

4.14-4.30 (2H, m, $-OCH_2-$)

4.70-4.89 (6H, m, $-CH_2ONO_2$, $-NH_2$, -NHCO-)

6.66 (1H, d-d, J=2.5Hz, 8Hz, H of the aromatic ring)

6.90 (1H, d, J=8Hz, H of the aromatic ring)

7.77 (1H, d, J=2.5Hz, H of the aromatic ring)

IR: $\nu_{max}^{KBr}$ $cm^{-1}$

1630 ($ONO_2$), 1690 (NHCO)

Exampl 17

3-Amino-2'-methoxy-5'-(2-nitroxyethoxy)propionanilide

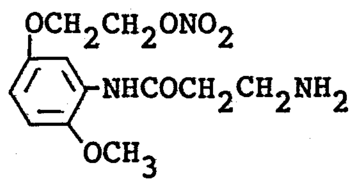

Yellow oily product

NMR: $\delta$ ($CDCl_3$)

2.50 (2H, t, J=6Hz, $-COCH_2-$)

3.10 (2H, t, J=6Hz, $-CH_2NH_2$)

3.82 (3H, s, $-OCH_3$)

4.20 (2H, t, J=4Hz, $-OCH_2-$)

4.77 (2H, t, J=4Hz, $-CH_2ONO_2-$)

6.52 (1H, d-d, J=3Hz, 8Hz, H of the aromatic ring)

6.77 (1H, d, J=8Hz, H of the aromatic ring)

8.10 (1H, d, J=3Hz, H of the aromatic ring)

IR: $\nu_{max}^{film}$ $cm^{-1}$

1630 ($ONO_2$), 1680 (NHCO)

Exampl 18

6-Amino-2'-methoxy-5'-(2-nitroxyethoxy)hexananilide

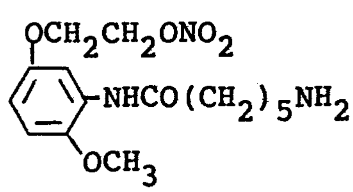

Pale yellow prism crystals

NMR: $\delta$ ($CDCl_3$)

1.20-1.96 (8H, m, $-NH_2$, $-(CH_2)_3-$)

2.39 (2H, t, J=7Hz, $-COCH_2-$)

2.70 (2H, t, J=6Hz, $-CH_2N<$)

3.81 (3H, s, $-OCH_3$)

4.10-4.29 (2H, m, $-OCH_2-$)

4.68-4.84 (2H, m, $-C\underline{H_2}ONO_2$)

6.53 (1H, d-d, J=2Hz, 8Hz, H of the aromatic ring)

6.75 (1H, d, J=8Hz, H of the aromatic ring)

7.75 (1H, br, s, $-N\underline{H}CO-$)

8.08 (1H, d, J=2Hz, H of the aromatic ring)

IR: $\nu_{max}^{film}$ $cm^{-1}$

1635 ($ONO_2$), 1680 (NHCO)

Exampl 19

2-Amino-2'-methoxy-5'-(2-nitroxyethoxy)propion-anilide·maleate

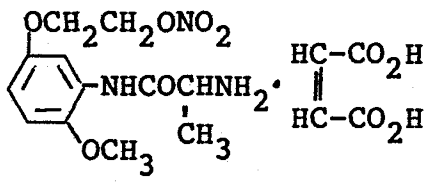

m.p. 125-127°C (ether-methanol), pale yellow fine needle-like crystals

NMR: $\delta$ ($CDCl_3$+$CD_3OD$))

1.60 (3H, d, J=8Hz, $-C\underline{H_3}$)

3.84 (3H, s, $-OC\underline{H_3}$)

4.10-4.40 (2H, m, $-OC\underline{H_2}-$)

6.24 (2H, s, $-C\underline{H}=C\underline{H}-$)

6.65 (1H, d-d, J=3Hz, 8Hz, H of the aromatic ring)

6.85 (1H, d, J=8Hz, H of the aromatic ring)

7.80 (1H, d, J=3Hz, H of the aromatic ring)

IR: $\nu_{max}^{KBr}$ $cm^{-1}$

1630 ($ONO_2$), 1640 (NHCO)

Exampl 20

2-Amino-2'-methyl-5'-(2-nitroxyethoxy)propion-anilide·maleate

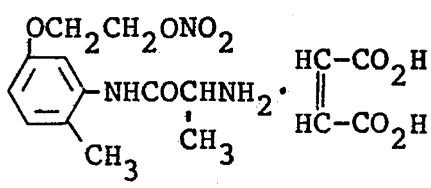

m.p. 113-118°C (acetone-ether), pale yellow powder

NMR: $\delta$ ($D_2O$)

1.73 (3H, d, J=8Hz, $-CH_3$)
2.17 (3H, s, $-CH_3$)
4.27-4.60 (2H, m, $-OCH_2-$)
4.60-5.00 (2H, m, $-CH_2ONO_2$)
6.28 (2H, s, -CH=CH-)
6.80-7.40 (3H, m, H of the aromatic ring)

IR: $\nu_{max}^{KBr}$ $cm^{-1}$

1630 ($ONO_2$), 1670 (NHCO)

Example 21

2-Amino-2'-methyl-6'-(2-nitroxyethoxy)propion-anilide

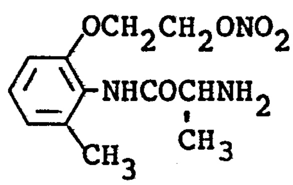

Pale yellow oily product

NMR: $\delta$ ($CDCl_3$)

1.42 (3H, d, J=6Hz, $-CH_3$)
1.72 (2H, s, $-NH_2$)
2.20 (3H, s, $-CH_3$)
3.67 (1H, q, J=6Hz, -COCH< )
4.13-4.36 (2H, m, $-OCH_2$)
4.71-4.92 (2H, m, $-CH_2ONO_2$)
6.70 (1H, d, J=8Hz, H of the aromatic ring)
6.86 (1H, d, J=8Hz, H of the aromatic ring)
7.09 (1H, t, J=8Hz, H of the aromatic ring)
8.74 (1H, br, s, -NHCO-)

IR: $\nu_{max}^{film}$ $cm^{-1}$

1635 ($ONO_2$), 1670 (NHCO), 3290 ($NH_2$)

Exampl 22

3-Amino-2'-methyl-5'-(2-nitroxyethoxy)butyr-anilide·hydrochloride

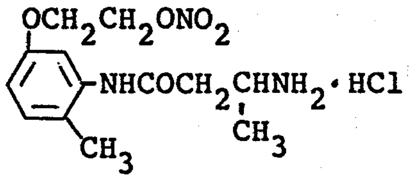

m.p. 162-164°C (methanol-ether), pale yellow fine needle-like crystals

NMR: δ ($D_2O$)

1.47 (3H, d, J=6Hz, $-CH_3$)

2.20 (3H, s, $-CH_3$)

2.92 (2H, d, J=6Hz, $-COCH_2-$)

3.70-4.00 (1H, m, $-CH<$)

4.25-4.43 (2H, m, $-OCH_2-$)

4.83-5.00 (2H, m, $-CH_2ONO_2$)

6.80-7.40 (3H, m, H of the aromatic ring)

IR: $\nu_{max}^{KBr}$ $cm^{-1}$

1630 ($ONO_2$), 1650 (NHCO)

Example 23

2-Amino-2'-methyl-5'-(3-nitroxypropoxy)propionanilide

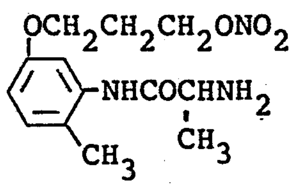

Pale yellow oily product

NMR: δ ($CDCl_3$)

1.43 (3H, d, J=6Hz, $-CH_3$)

2.20 (3H, s, $-CH_3$)

2.00-2.30 (2H, m, $-CH_2-$)

3.63 (1H, q, J=6Hz, $-CH<$)

4.05 (2H, t, J=6Hz, $-OCH_2-$)

4.63 (2H, t, J=6Hz, $-CH_2ONO_2$)

6.55 (1H, d-d, J=3Hz, 8Hz, H of the aromatic ring)

7.02 (1H, d, J=8Hz, H of the aromatic ring)

7.85 (1H, d, J=3Hz, H of the aromatic ring)

IR: $\nu_{max}^{film}$ $cm^{-1}$

1620 ($ONO_2$), 1670 (NHCO)

Example 24

2-Amino-2'-fluoro-5'-(2-nitroxyethoxy)propion-anilide

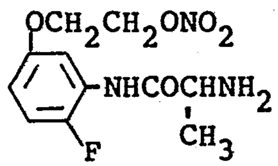

Reddish brown viscous oily product

NMR: $\delta$ ($CDCl_3$)

1.43 (3H, d, J=7Hz, $-C\underline{H_3}$)

1.66 (2H, s, $-N\underline{H_2}$)

3.64 (1H, q, J=7Hz, $-COC\underline{H}$)

4.16-4.32 (1H, m, $-O\underline{CH_2}-$)

4.72-4.88 (2H, m, $-\underline{CH_2}ONO_2$)

6.44-6.66 (1H, m, H of the aromatic ring)

6.98 (1H, t, J=10Hz, H of the aromatic ring)

8.07 (1H, d-d, J=3Hz, 7Hz, H of the aromatic ring)

IR: $\nu_{max}^{film}$ $cm^{-1}$

1635 ($ONO_2$), 1680 (NHCO), 3290 ($NH_2$)

Example 25

2-Amino-2'-chloro-5'-(2-nitroxyethoxy)propion-anilide

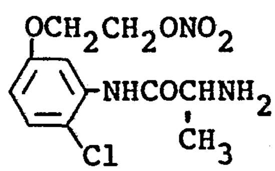

Pale yellow oily product

NMR: $\delta$ ($CDCl_3$)

1.45 (3H, d, J=7Hz, $-C\underline{H_3}$)

1.76 (2H, br, s, $-N\underline{H_2}$)

3.50-3.82 (2H, m, $-\underline{CHN}<$)

4.20-4.36 (2H, m, $-O\underline{CH_2}-$)

4.73-4.92 (2H, m, $-CH_2ONO_2$)
6.60 (1H, d-d, J=3Hz, 8Hz, H of the aromatic ring)
7.26 (1H, d, J=8Hz, H of the aromatic ring)
8.20 (1H, d, J=3Hz, H of the aromatic ring)
10.24 (1H, m, -NHCO-)

IR: $\nu_{max}^{film}$ $cm^{-1}$
1640 ($ONO_2$), 1690 (NHCO), 3270 ($NH_2$)

Example 26

2-Amino-2'-(2,2,2-trifluoroethoxy)-5'-(2-nitroxyethoxy)propionanilide·maleate

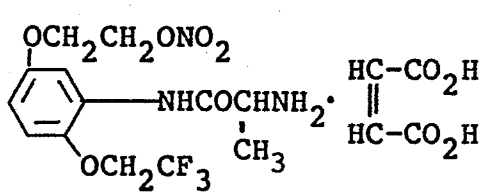

m.p. 153-154°C (methanol-ether), white powder

NMR: $\delta$ ($D_2O$)
1.73 (3H, d, J=6Hz, $-CH_3$)
4.28-4.40 (2H, m, $-OCH_2-$)
4.57 (2H, q, J=8Hz, $-CH_2CF_3$)
4.83-5.00 (2H, m, $-CH_2ONO_2$)
6.28 (2H, s, -CH=CH-)
6.90 (1H, d-d, J=3Hz, 8Hz, H of the aromatic ring)
7.13 (1H, d, J=8Hz, H of the aromatic ring)
7.25 (1H, d, J=3Hz, H of the aromatic ring)

IR: $\nu_{max}^{KBr}$ $cm^{-1}$
1620 ($ONO_2$), 1680 (NHCO)

Example 27

2-Amino-2'-methyl-5'-(2-nitroxyethoxy)butyranilide·hydrochloride

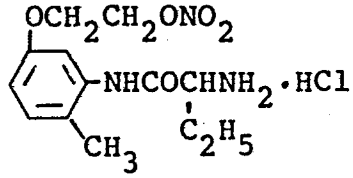

m.p. 135-137°C (methanol-ether), pale yellow fine needle-like crystals

NMR: $\delta$ ($D_2O$)

1.13 (3H, t, J=6Hz, $-CH_3$)

1.95-2.30 (2H, m, $-CH_2-$)

2.17 (3H, s, $-CH_3$)

4.30-4.50 (2H, m, $-OCH_2-$)

4.83-5.00 (2H, m, $-CH_2ONO_2$)

6.80-7.40 (3H, m, H of the aromatic ring)

IR: $\nu_{max}^{KBr}$ $cm^{-1}$

1630 ($ONO_2$), 1660 (NHCO)

Example 28

2-Amino-3',5'-dimethyl-4'-(2-nitroxyethoxy)-propionanilide·hydrochloride

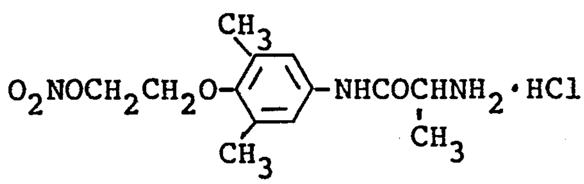

m.p. 190-193°C (decomposition)(ethanol), colorless crystals

NMR: $\delta$ (DMSO-$d_6$)

1.46 (3H, d, J=6Hz, $-CH_3$)

2.18 (6H, s, $-CH_3$)

3.34 (2H, br, s, $-NH_2$)

3.86-4.24 (3H, m, -COCH, $-OCH_2-$)

4.80-4.96 (2H, m, $-CH_2ONO_2$)

7.30 (2H, s, H of the aromatic ring)

8.22-8.54 (2H, m, -NHCO-, HCl)

IR: $\nu_{max}^{KBrm}$ $cm^{-1}$

1630 ($ONO_2$), 1690 (NHCO), 3230 ($NH_2$)

Example 29

2-Amino-2',6'-dimethyl-3'-(2-nitroxyethoxy)-propionanilide·maleate

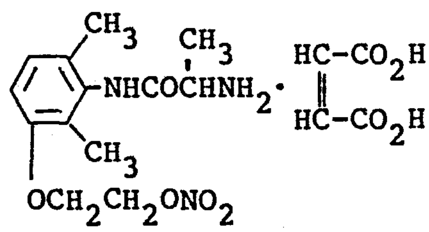

m.p. 153-155°C (ethanol-ether), colorless prism crystals

NMR: $\delta$($CD_3OD$)

1.68 (3H, d, J=6Hz, $-CH_3$)

2.06 (3H, s, $-CH_3$)

2.14 (3H, s, $-CH_3$)

4.12-4.38 (3H, m, -COCH, $-OCH_2-$)

4.70-5.04 (7H, m, $-CH_2ONO_2$, -NHCO-, $-NH_2$, -COOH)

6.22 (2H, s, -CH=CH-)

6.82 (1H, d, J=8Hz, H of the aromatic ring)

7.06 (1H, d, J=8Hz, H of the aromatic ring)

IR: $\nu_{max}^{KBr}$ $cm^{-1}$

1635, ($ONO_2$, COOH), 1670 (NHCO)

Example 30

2-Amino-2'-methyl-5'-(2-nitroxypropoxy)-propionanilide

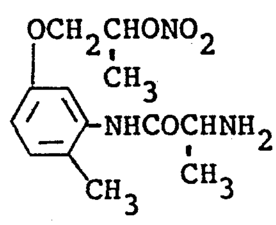

Pale yellow oily product

NMR: $\delta$($CDCl_3$)

1.43 (6H, d, J=6Hz, $-CH_3$)

2.19 (3H, s, $-CH_3$)

3.62 (1H, q, J=6Hz, -COC<u>H</u>< )

4.05 (2H, d, J=6Hz, $-OCH_2-$)

5.30-5.60 (1H, m, >C<u>H</u>$ONO_2$)

6.60 (1H, d-d, J=3Hz, 8Hz, H of the aromatic ring)

7.03 (1H, d, J=8Hz, H of the aromatic ring)

7.90 (1H, d, J=3Hz, H of the aromatic ring)

IR: $\nu_{max}^{film}$ $cm^{-1}$

1620 ($ONO_2$), 1670 (NHCO)

Mass: m/e

297 ($M^+$), 280 ($M^+-NH_3$)

<u>Example 31</u>

2-Amino-2'-methyl-6'-(2-nitroxypropoxy)-propionanilide

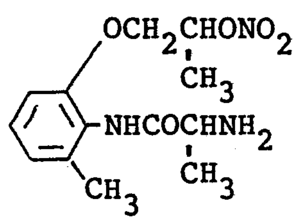

Pale yellow oily product

NMR: $\delta$ ($CDCl_3$)

1.47 (6H, d, J=6Hz, $-CH_3$)

1.70 (2H, br, s, $-NH_2$)

2.25 (3H, s, $-CH_3$)

3.60-3.83 (1H, m, >C<u>H</u>-)

4.05-4.20 (2H, m, $-OCH_2-$)

5.30-5.80 (1H, m, >C<u>H</u>$ONO_2$)

6.60-7.40 (2H, m, H of the aromatic ring)

7.16 (1H, t, J=8Hz, H of the aromatic ring)

8.84 (1H, m, -N<u>H</u>CO-)

IR: $\nu_{max}^{film}$ $cm^{-1}$

1625 ($ONO_2$), 1670 (NHCO), 3290 ($NH_2$)

<u>Example 32</u>

2-Amino-2',6'-dimethyl-3'-(2-nitroxypropoxy)-propionanilide

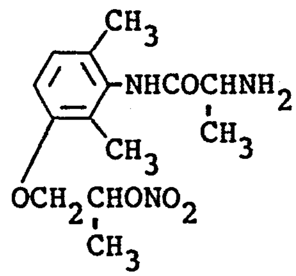

m.p. 78-79°C, colorless prism crystals

NMR: $\delta$ ($CDCl_3$)

1.42 (6H, t, J=6Hz, -$CH_3$)
1.79 (2H, br, s, -$NH_2$)
2.00 (3H, s, -$CH_3$)
2.09 (3H, s, -$CH_3$)
3.58 (1H, quintet, J=6Hz, >CHN<)
4.01 (2H, d, J=5Hz, -$OCH_2$-)
5.30-5.64 (1H, m, >$CHONO_2$)
6.60 (1H, d, J=8Hz, H of the aromatic ring)
6.95 (1H, d, J=8Hz, H of the aromatic ring)
8.81 (1H, br, s, -NHCO-)

IR: $\nu_{max}^{KBr}$ $cm^{-1}$

1630 ($ONO_2$), 1665 (NHCO), 3265 ($NH_2$)

Example 33

3-(2-Chlorobenzoylamino)-2'-methoxy-5'-(2-nitroxyethoxy)propionanilide

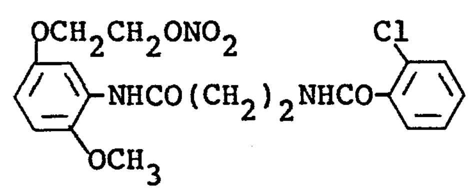

m.p. 138-139°C (n-hexane-acetone), pale yellow prism crystals

NMR: $\delta$ ($CDCl_3$)

2.77 (2H, t, J=6Hz, -$COCH_2$-)
3.80 (3H, s, -$OCH_3$)
4.20 (2H, t, J=4Hz, -$OCH_2$-)
4.77 (2H, t, J=4Hz, -$CH_2ONO_2$)
6.50-8.00 (7H, m, H of the aromatic ring)

IR: $\nu_{max}^{KBr}$ $cm^{-1}$

1630 ($ONO_2$), 1650-1660 (NHCO)

Example 34

6-(2-Chlorobenzoylamino)-2'-methoxy-5'-(2-nitroxyethoxy)hexananilide

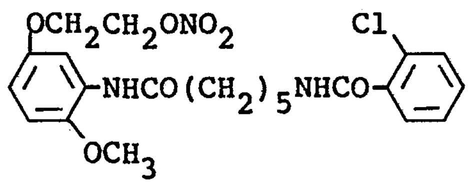

m.p. 106-107°C (ether-ethyl acetate), colorless fine needle-like crystals

NMR: $\delta$ ($CDCl_3$)

1.34-1.94 (6H, m, $-(CH_2)_3-$)

2.39 (2H, t, J=7Hz, $-COCH_2-$)

3.45 (2H, q, J=6Hz, $-CH_2N<$ )

3.81 (3H, s, $-OCH_3$)

4.12-4.29 (2H, m, $-OCH_2-$)

4.67-4.85 (2H, m, $-CH_2ONO_2$)

6.00-6.40 (1H, m, -NHCO-)

6.51 (1H, d-d, J=2Hz, 8Hz, H of the aromatic ring)

6.73 (1H, d, J=8Hz, H of the aromatic ring)

7.18-7.84 (4H, m, -NHCO-, H of the aromatic ring)

8.06 (1H, d, J=2Hz, H of the aromatic ring)

IR: $\nu_{max}^{KBr}$ $cm^{-1}$

1635 ($ONO_2$), 1660 (NHCO)

Example 35

6-(3-Phenylpropionylamino)-2'-methoxy-5'-(2-nitroxyethoxy)hexananilide

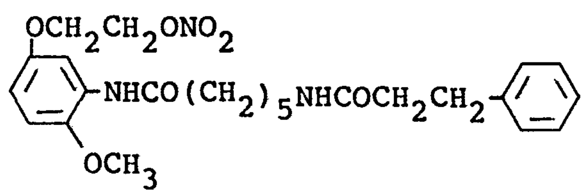

m.p. 107-108°C (decomposition)(n-hexane-ethyl acetate), colorlesss fine needle-like crystals

NMR: δ ($CDCl_3$)

1.20-1.92 (6H, m, $-(CH_2)_3-$)

2.24-2.60 (4H, m, $-COCH_2-$)

2.94 (2H, t, J=8Hz, $-CH_2-$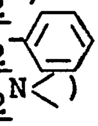)

3.20 (2H, q, J=6Hz, $-CH_2N<$)

3.80 (3H, s, $-OCH_3$)

4.11-4.28 (2H, m, $-OCH_2-$)

4.64-4.80 (2H, m, $-CH_2ONO_2$)

5.40-5.80 (1H, m, -NHCO-)

6.52 (1H, d-d, J=2Hz, 8Hz, H of the aromatic ring)

6.75 (1H, d, J=8Hz, H of the aromatic ring)

7.16 (5H, s, H of the aromatic ring)

7.74 (1H, br, s, -NHCO-)

8.06 (1H, d, J=2Hz, H of the aromatic ring)

IR: $\nu_{max}^{KBr}$ $cm^{-1}$

1640 ($ONO_2$), 1670 (NHCO)

Example 36

2-(2-Chlorobenzoylamino)-2'-methoxy-5'-(2-nitroxyethoxy)propionanilide

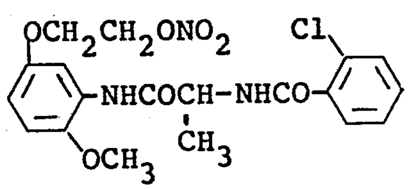

m.p. 110-112°C (n-hexane-acetone), pale yellow powder

NMR: δ($CDCl_3$)

1.58 (3H, d, J=6Hz, $-CH_3$)

3.82 (3H, s, $-OCH_3$)

4.10-4.30 (2H, m, $-OCH_2-$)

4.65-4.85 (2H, m, $-CH_2ONO_2$)

6.45-8.00 (7H, m, H of the aromatic ring)

IR: $\nu_{max}^{KBr}$ $cm^{-1}$

1630 ($ONO_2$), 1640-1680 (NHCO)

Example 37

2-Amino-2',6'-dimethyl-4'-(2-fluoroethoxy)-propionanilide·hydrochloride

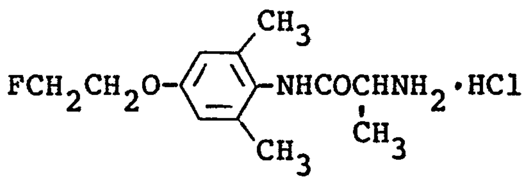

m.p. 215-218°C (decomposition), colorless crystals

NMR: $\delta$ ($CD_3OD$)

1.68 (3H, d, J=7Hz, $-CH_3$)

2.18 (6H, s, $-CH_3$)

3.98-4.52 (4H, m, $-OCH_2-$, -CHF, >CHN<)

4.56 (3H, s, $-NH_2$, -NHCO, HCl)

4.88-5.00 (1H, m, CHF-)

6.69 (2H, s, H of the aromatic ring)

IR: $\nu_{max}^{KBr}$ $cm^{-1}$

1670 (NHCO)

Mass: m/e

254 ($M^+$)

Example 38

2-Amino-2'-methyl-5'-(2,2,2-trifluoroethoxy)-propionanilide

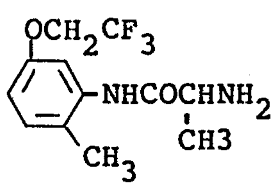

m.p. 90-92°C (ether-n-hexane), pale brown prism crystals

NMR: $\delta$ ($CDCl_3$)

1.45 (3H, d, J=6.5Hz, $-CH_3$)

1.66 (2H, br, s, $-NH_2$)

2.23 (3H, s, $-CH_3$)

3.65 (1H, q, J=6.5Hz, >CHN<)

4.35 (2H, q, J=8Hz, $-OCH_2-$)

6.63 (1H, d-d, J=2.5Hz, 8Hz, H of the aromatic ring)

7.07 (1H, d, J=8Hz, H of the aromatic ring)

7.97 (1H, d, J=2.5Hz, H of the aromatic ring)

9.66 (1H, br, s, -NHCO-)

IR: $\nu_{max}^{KBr}$ $cm^{-1}$

1675 (NHCO), 3240, 3360 ($NH_2$)

Example 39

2-Amino-2'-methyl-6'-(2,2,2-trifluoroethoxy)-propionanilide

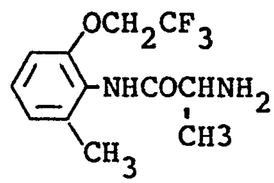

m.p. 83-84°C (ether-n-hexane), colorless prism crystals

NMR: $\delta$ ($CDCl_3$)

1.44 (3H, d, J=6Hz, $-CH_3$)

1.62 (2H, s, $-NH_2$)

2.23 (3H, s, $-CH_3$)

3.69 (1H, q, J=7Hz, >CHN<)

4.32 (2H, q, J=8Hz, $-OCH_2-$)

6.72 (1H, d, J=8Hz, H of the aromatic ring)

6.94 (1H, d, J=8Hz, H of the aromatic ring)

7.13 (1H, t, J=8Hz, H of the aromatic ring)

8.63-9.03 (1H, m, -NHCO-)

IR: $\nu_{max}^{KBr}$ $cm^{-1}$

1655 (NHCO), 3240 ($NH_2$)

Example 40

3-Amino-2'-methyl-5'-(2,2,2-trifluoroethoxy)-butyranilide·hydrochloride

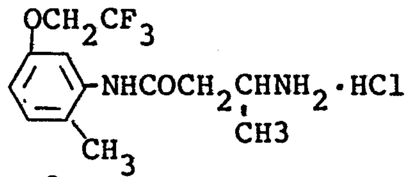

m.p. 202-204°C (methanol-ether), colorless powder

NMR: δ ($D_2O$)

0.73 (3H, d, J=7Hz, $-CH_3$)

2.17 (3H, s, $-CH_2$)

2.88 (3H, d, J=7Hz, $-COCH_2-$)

3.67-4.05 (1H, m, >CHN< )

4.56 (2H, q, J=8Hz, $-CH_2CF_3$)

6.80-7.40 (3H, m, H of the aromatic ring)

IR: $\nu_{max}^{KBr}$ $cm^{-1}$

1660 (NHCO), 3300 ($NH_2$)

Example 41

2-Amino-2',6'-dimethyl-4'-(2,2,2-trifluoroethoxy)propionanilide·hydrochloride

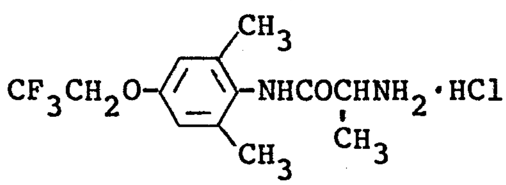

m.p. 236-239°C (decomposition)(ethanol-ether), colorless mica-like crystals

NMR: δ (DMSO-$d_6$)

1.53 (3H, d, J=7Hz, $-CH_3$)

2.12 (6H, s, $-CH_3$)

3.29 (2H, s, $-NH_2$)

4.12 (1H, q, J=7Hz, >CHN< )

4.68 (2H, q, J=8Hz, $-CH_2CF_3$)

6.79 (2H, s, H of the aromatic ring)

8.37 (2H, br, s, -NHCO-, HCl)

IR: $\nu_{max}^{KBr}$ $cm^{-1}$

1665 (NHCO), 3250, 3400 ($NH_2$)

Example 42

1-[2-Methoxy-5-(2,2,2-trifluoroethoxy)phenoxy]-2-propanamine • hydrochloride

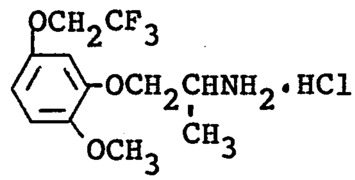

m.p. 135-136°C (methanol-ether), pale brown mica-like crystals

NMR: δ($D_2O$)

1.52 (3H, d, J=6Hz, $-CH_3$)

3.92 (3H, s, $-OCH_3$)

4.58 (2H, q, J=8Hz, $-OCH_2CF_3$)

6.65-7.20 (3H, m, H of the aromatic ring)

IR: $\nu_{max}^{KBr}$ $cm^{-1}$

1510, 1610 (aromatic ring), 3500 ($NH_2$)

Example 43

1-[2-Methyl-5-(2-nitroxyethoxy)phenoxy]-2-propanamine • maleate

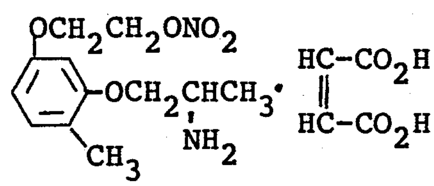

m.p. 94-96°C (methanol-ether), colorless needle-like crystals

NMR: δ($D_2O$)

1.48 (3H, d, J=6Hz, $-CH_3$)

2.15 (3H, s, $-CH_3$)

4.23-4.43 (2H, m, $-OCH_2-$)

4.80-5.00 (2H, m, $-CH_2ONO_2$)

6.25 (2H, s, $-CH=CH-$)

6.65-7.25 (3H, m, H of the aromatic ring)

IR: $\nu_{max}^{KBr}$ $cm^{-1}$

1620 ($ONO_2$)

Example 44

1-[5-Nitroxyethoxy-2-(2,2,2-trifluoroethoxy)-phenoxy]-2-propanamine

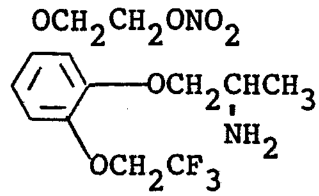

Yellow oily product

NMR: $\delta$($CDCl_3$)

1.18 (3H, d, J=6Hz, $-CH_3$)

4.30 (2H, q, J=8Hz, $-OCH_2CF_3$)

4.77 (2H, t, J=6Hz, $-CH_2ONO_2$)

6.30-6.91 (3H, m, H of the aromatic ring)

IR: $\nu_{max}^{film}$ $cm^{-1}$

1620 ($ONO_2$)

Example 45

2-(2-Aminopropoxy)-4-(2-nitroxyethoxy)acetophenone

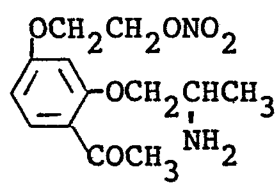

Pale yellow oily product

NMR: $\delta$($CDCl_3$)

1.30 (3H, d, J=7Hz, $-CH_3$)

2.38 (3H, s, $-COCH_3$)

4.75-4.95 (2H, m, $-CH_2ONO_2$)

6.45-7.45 (3H, m, H of the aromatic ring)

IR: $\nu_{max}^{film}$ $cm^{-1}$

1630 ($ONO_2$), 1660 ($COCH_3$)

Mass: m/e

280 ($M^+-H_2O$)

Example 46

1-[2,6-Dimethyl-4-(2-nitroxyethoxy)phenoxy]-2-propanamine • hydrochloride

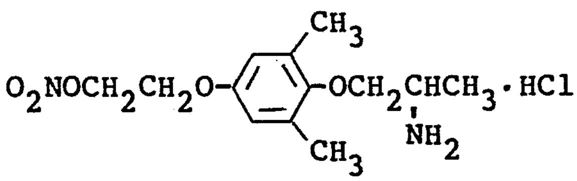

m.p.: 184-185°C (methanol-ether), slightly brown needle-like crystals

NMR: δ($D_2O$)

1.56 (3H, d, J=6Hz, $-CH_3$)
2.35 (6H, s, $-CH_3$)
4.25-4.45 (2H, m, $-OCH_2-$)
4.90-5.08 (2H, m, $-CH_2ONO_2$)
6.80 (2H, s, H of the aromatic ring)

IR: $\nu_{max}^{KBr}$ $cm^{-1}$
1630 ($ONO_2$)

Example 47

1-[2-Methoxy-4-(2-nitroxyethoxy)phenoxy]-2-propanamine

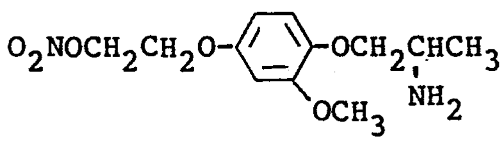

Pale yellow oily product

NMR: δ($CDCl_3$)

1.13 (3H, d, J=6Hz, $-CH_3$)
3.82 (3H, s, $-OCH_3$)
4.10-4.30 (2H, m, $-OCH_2-$)
4.65-4.90 (2H, m, $-CH_2ONO_2$)
6.30-6.80 (3H, m, H of the aromatic ring)

IR: $\nu_{max}^{film}$ $cm^{-1}$
1630 ($ONO_2$)

Mass: m/e
286 ($M^+$)

Example 48

1-[2-Fluoro-4-(2-nitroxyethoxy)phenoxy]-2-propanamine

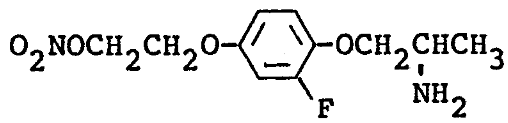

Slightly pale yellow oily product

NMR: $\delta$ ($CDCl_3$)

1.15 (3H, d, J=6Hz, $-CH_3$)

1.54 (3H, s, $-NH_2$)

3.16-3.58 (1H, m, >CHN<)

3.58-4.00 (2H, m, $-OCH_2-$)

4.12-4.36 (2H, m, $-OCH_2CH_2-$)

4.72-4.92 (2H, m, $-CH_2ONO_2$)

6.48-7.04 (3H, m, H of the aromatic ring)

IR: $\nu_{max}^{film}$ $cm^{-1}$

1635 ($ONO_2$)

Mass: m/e

274 ($M^+$)

Example 49

1-[2-Methoxy-5-(2-nitroxyethoxy)phenoxy]-2-butanamine

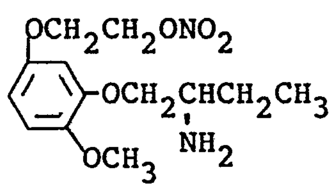

Pale yellow oily product

NMR: $\delta$ ($CDCl_3$)

1.00 (3H, t, J=7Hz, $-CH_3$)

1.30-1.80 (2H, m, $-CH_2-$)

3.80 (3H, s, $-OCH_3$)

4.18 (2H, t, J=6Hz, $-OCH_2CH_2-$)

4.78 (2H, t, J=6Hz, $-CH_2ONO_2$)

6.35-6.80 (3H, m, H of the aromatic ring)

IR: $\nu_{max}^{film}$ $cm^{-1}$
1630 ($ONO_2$)

Mass: m/e
300 ($M^+$)

Example 50

1-[2-Methoxy-5-(2-nitroxyethoxy)phenoxy]-2-piperidinopropane

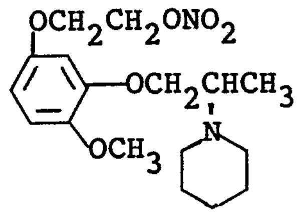

Pale brown oily product

NMR: $\delta$($CDCl_3$)
1.21 (3H, d, J=6Hz, $-CH_3$)
1.35-1.80 (6H, m, 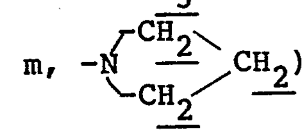)
2.60-2.80 (4H, m, 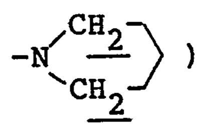)
3.00-3.25 (1H, m, >CHN<)
3.80 (3H, s, $-OCH_3$)
4.75-4.90 (2H, m, $-CH_2ONO_2$)
6.40-6.80 (3H, m, H of the aromatic ring)

IR: $\nu_{max}^{film}$ $cm^{-1}$
1630 ($ONO_2$)

Mass: m/e
354 ($M^+$)

Example 51

1-[3-Fluoro-4-(2,2,2-trifluoroethoxy)-phenoxy]-2-propanamine · hydrochloride

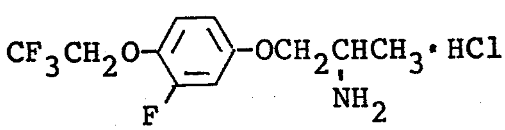

m.p. 160-162°C (ethanol-ether), colorless prism crystals

NMR: δ ($CD_3OD$)

1.42 (3H, d, J=6Hz, $-C\underline{H_3}$)

3.50-3.84 (1H, m, $>C\underline{H}N<$)

3.84-4.28 (2H, m, $-OCH_2-$)

4.48 (2H, q, J=8Hz, $-O\underline{CH_2}CF_3$)

6.68-7.24 (3H, m, H of the aromatic ring)

IR: $\nu_{max}^{KBr}$ $cm^{-1}$

1600 (aromatic ring)

Example 52

1-[2-methoxy-3,5-bis(2,2,2-trifluoroethoxy)-phenoxy]-2-propanamine

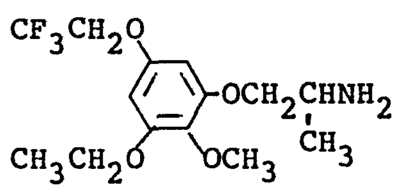

Pale yellowish brown oily product

NMR: δ ($CDCl_3$)

1.19 (3H, d, J=6Hz, $-C\underline{H_3}$)

1.53 (2H, s, $N\underline{H_2}$)

3.18-3.56 (1H, m, $-C\underline{H}-$)

3.60-4.00 (5H, m, $-OC\underline{H_3}$, $-OC\underline{H_2}-$)

4.28-4.40 (2H, q, J=8Hz, $-OC\underline{H_2}CF_3$)

6.21-6.32 (1H, d, J=3Hz, H of the aromatic ring)

IR: $\nu_{max}^{film}$ $cm^{-1}$

1600 (aromatic ring)

Example 53

2-Amino-2',6'-dimethyl-4'-(3-nitroxypropoxy)-propionanilide·hydrochloride

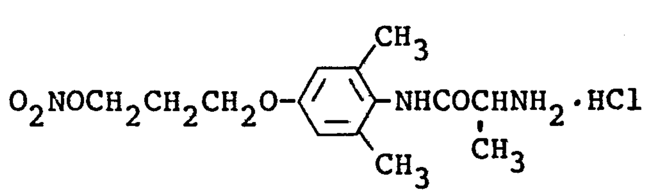

m.p. 172-174°C, colorless needle-like crystals

NMR: $\delta$ ($CD_3OD$)

1.72 (3H, d, J=7Hz, $-C\underline{H_3}$)

2.20 (2H, m, $-C\underline{H_2}-$)

2.24 (6H, s, $-C\underline{H_3}$)

4.08 (2H, t, J=6Hz, $-OC\underline{H_2}-$)

4.26 (1H, q, J=7Hz, $-COC\underline{H}<$)

4.70 (2H, t, J=6Hz, $-C\underline{H_2}ONO_2$)

6.68 (2H, s, H of the aromatic ring)

IR: $\nu_{max}^{KBr}$ $cm^{-1}$

1630 ($ONO_2$), 1660 (NHCO)

What we claim is:

1. An aminoalkyl-substituted benzene derivative represented by the formula

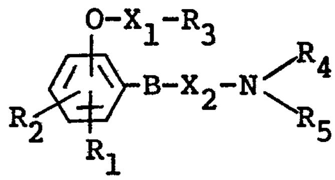

.....(I)

wherein $R_1$ represents a member selected from the class consisting of a hydrogen atom, lower alkyl groups, lower alkoxy groups, lower aliphatic acyl groups, halogen atoms and a trifluoroethoxy group; $R_2$ represents a hydrogen atom or a lower alkyl group; $R_3$ represents $-ONO_2$, $-CF_3$ or F; $R_4$ and $R_5$, independently from each other, represent a member selected from the class consisting of a hydrogen atom, lower alkyl groups, halogen-substituted benzoyl groups, phenyl-substituted lower aliphatic acyl groups and a pyridinecarbonyl group, or $R_4$ and $R_5$ may form, together with the nitrogen atom to which they are bonded, a 6-membered hetero ring which may contain another hetero atom selected from O and N; $X_1$ and $X_2$, independently from each other, represents an alkylene group having 1 to 8 carbon atoms; and B represents -O- or -NHCO- in which the nitrogen atom of -NHCO- is bonded to a carbon atom of the benzene ring;

and an acid addition salt thereof.

2. The compound and its acid addition salt in claim 1 wherein $R_1$ is a member selected from the class consisting of a hydrogen atom, alkyl groups having 1 to 4 carbon atoms, alkoxy groups having 1 to 4 carbon atoms, aliphatic acyl groups having 2 to 4 carbon toms, halogen atoms, and a trifluoroethoxy group; $R_2$ reprsents a hydrogen atom or an alkyl group having 1 to 4 carbon atoms, $R_3$ represents

$-ONO_2$, $-CF_3$ or F; $R_4$ and $R_5$, independently from each other, represent a member selected from the class consisting of a hydrogen atom, alkyl groups having 1 to 4 carbon atoms, halogen-substituted benzoyl groups, phenyl-substituted $C_2$-$C_4$ aliphatic acyl groups and a pyridinecarbonyl group or $R_4$ and $R_5$ may, together with the nitrogen atom to which they are bonded, form a piperidino, piperazino or morpholino ring which may be substituted by a lower alkyl group; $X_1$ and $X_2$, independently from each other, represent an alkylene group having 1 to 6 carbon atoms; and B represents -O- or -NHCO- in which the nitrogen atom of -NHCO- is bonded to a carbon atom of the benzene ring.

3. 2-Dimethylamino-2'-methoxy-5'-(2-nitroxyethoxy)-acetanilide and its acid addition salt.

4. 3-Amino-2'-methoxy-5'-(2-nitroxyethoxy)-butyranilide and its acid addition salt.

5. 3-Amino-2'-methoxy-5'-(2,2,2-trifluoroethoxy)-butyranilide and its acid addition salt.

6. 1-[2-Methoxy-5-(2-nitroxyethoxy)phenoxy]-2-propanamine and its acid addition salt.

7. 1-[2-Methoxy-5-(2-nitroxyethoxy)phenoxy]-3-butanamine and its acid addition salt.

8. 2-Amino-2',6'-dimethyl-4'-(2-nitroxyethoxy)-propionanilide and its acid addition salt.

9. 2-Amino-2'-methoxy-5'-(2-nitroxypropoxy)-propionanilide and its acid addition salt.

10. 2-(2-Pyridinecarbonylamino)-2'-methoxy-5'-(2-nitroxyethoxy)-acetanilide and its acid addition salt.

11. 3-Amino-5'-(2-fluoroethoxy)-2'-methoxybutyranilide and its acid addition salt.

12. 2-Amino-2',6'-dimethyl-4'-(6-nitroxyhexyloxy)-propionanilide and its acid addition salt.

13. 3-Morpholino-2'-methoxy-5'-(2-nitroxyethoxy)-propionanilide and its acid addition salt.

14. 2-Morpholino-2'-methoxy-5'-(2-nitroxyethoxy)-acetanilide and its acid addition salt.

15. 3-Piperidino-2'-methoxy-5'-(2-nitroxyethoxy)-propionanilide and its acid addition salt.
16. 2'-Methoxy-3-(4-methylpiperazino)-5'-(2-nitroxyethoxy)-propionanilide and its acid addition salt.
17. 2-(Dimethylamino)-2'-methoxy-4'-(2-nitroxyethoxy)-acetanilide and its acid addition salt.
18. 2-Amino-2'-methoxy-5'-(2-nitroxyethoxy)-acetanilide and its acid addition salt.
19. 3-Amino-2'-methoxy-5'-(2-nitroxyethoxy)-propionanilide and its acid addition salt.
20. 6-Amino-2'-methoxy-5'-(2-nitroxyethoxy)-hexananilide and its acid addition salt.
21. 2-Amino-2'-methoxy-5'-(2-nitroxyethoxy)-propionanilide and its acid addition salt.
22. 2-Amino-2'-methyl-5'-(2-nitroxyethoxy)-propionanilide and its acid addition salt.
23. 2-Amino-2'-methyl-6'-(2-nitroxyethoxy)-propionanilide and its acid addition salt.
24. 3-Amino-2'-methyl-5'-(2-nitroxyethoxy)-butyranilide and its acid addition salt.
25. 2-Amino-2'-methyl-5'-(3-nitroxypropoxy)-propionanilide and its acid addition salt.
26. 2-Amino-2'-fluoro-5'-(2-nitroixyethoxy)-propionanilide and its acid addition salt.
27. 2-Amino-2'-chloro-5'-(2-nitroxyethoxy)-propionanilide and its acid addition salt.
28. 2-Amino-2'-(2,2,2-trifluoroethoxy)-5'-(2-nitroxyethoxy)-propionanilide and its acid addition salt.
29. 2-Amino-2'-methyl-5'-(2-nitroxyethoxy)-butyranilide and its acid addition salt.
30. 2-Amino-3',5'-dimethyl-4'-(2-nitroxyethoxy)-propionanilide and its acid addition salt.
31. 2-Amino-2',6'-dimethyl-3'-(2-nitroxyethoxy)-propionanilide and its acid addition salt.
32. 2-Amino-2'-methyl-5'-(2-nitroxypropoxy)-propionanilide and its acid addition salt.

33. 2-Amino-2'-methyl-6'-(2-nitroxypropoxy)-propionanilide and its acid addition salt.
34. 2-Amino-2',6'-dimethyl-3'-(2-nitroxypropoxy)-propionanilide and its acid addition salt.
35. 3-(2-Chlorobenzoylamino)-2'-methoxy-5'-(2-nitroxyethoxy)-propionanilide and its acid addition salt.
36. 6-(2-Chlorobenzoylamino)-2'-methoxy-5'-(2-nitroxyethoxy)-hexananilide and its acid addition salt.
37. 6-(3-Phenylpropionylamino)-2'-methoxy-5'-(2-nitroxyethoxy)-hexananilide and its acid addition salt.
38. 2-(2-Chlorobenzoylamino)-2'-methoxy-5'-(2-nitroxyethoxy)-propionanilide and its acid addition salt.
39. 2-Amino-2',6'-dimethyl-4'-(2-fluoroethoxy)-propionanilide and its acid addition salt.
40. 2-Amino-2'-methyl-5'-(2,2,2-trifluoroethoxy)-propionanilide and its acid addition salt.
41. 2-Amino-2'-methyl-6'-(2,2,2-trifluoroethoxy)-propionanilide and its acid addition salt.
42. 3-Amino-2'-methyl-5'-(2,2,2-trifluoroethoxy)-butyranilide and its acid addition salt.
43. 2-Amino-2',6'-dimethyl-4'-(2,2,2-trifluoroethoxy)-propionanilide and its acid addition salt.
44. 1-[2-Methoxy-5-(2,2,2-trifluoroethoxy)phenoxy]-2-propanamine and its acid addition salt.
45. 1-[2-Methyl-5-(2-nitroxyethoxy)phenoxy]-2-propanamine and its acid addition salt.
46. 1-[5-(Nitroxyethoxy)-2-(2,2,2-trifluoroethoxy)-phenoxy]-2-propanamine and its acid addition salt.
47. 2-(2-Aminopropoxy)-4-(2-nitroxyethoxy)acetophenone and its acid addition salt.
48. 1-[2,6-Dimethyl-4-(2-nitroxyethoxy)phenoxy]-2-propanamine and its acid addition salt.
49. 1-[2-Methoxy-4-(2-nitroxyethoxy)phenoxy]-2-propanamine and its acid addition salt.
50. 1-[2-Fluoro-4-(2-nitroxyethoxy)phenoxy]-2-propanamine and its acid addition salt.

51. 1-[2-Methoxy-5-(2-nitroxyethoxy)pohenoxy]-2-butanamine and its acid addition salt.

52. 1-[2-Methoxy-5-(2-nitroxyethoxy)phenoxy]-2-piperidinepropane and its acid addition salt.

53. 1-[3-Fluoro-4-(2,2,2-trifluoroethoxy)phenoxy]-2-propanamine and its acid addition salt.

54. 1-[2-Methoxy-3,5-bis(2,2,2-trifluoroethoxy)-phenoxy]- 2-propanamine and its acid addition salt.

55. 2-Amino-2',6'-dimethyl-4'-(3-nitroxypropoxy)-propion- anilide and its acid addition salt.

56. A process for producing an aminoalkyl-substituted benzene derivative represented by the following formula (1)

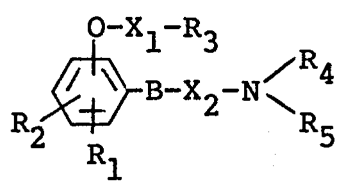

.....(I)

wherein $R_1$ represents a member selected from the class consisting of a hydrogen atom, lower alkyl groups, lower alkoxy groups, lower aliphatic acyl groups, halogen atoms and a trifluoroethoxy group; $R_2$ represents a hydrogen atom or a lower alkyl group; $R_3$ represents $-ONO_2$, $-CF_3$ or F; $R_4$ and $R_5$, independently from each other, represent a member selected from the class consisting of a hydrogen atom, lower alkyl groups, halogen-substituted benzoyl groups, phenyl-substituted lower aliphatic acyl groups and a pyridinecarbonyl group, or $R_4$ and $R_5$ may form, together with the nitrogen atom to which they are bonded, a 6-membered hetero ring which may contain another hetero atom selected from O and N; $X_1$ and $X_2$, independently from each other, represents an alkylene group having 1 to 8 carbon atoms; and B represents -O- or -NHCO- in which the nitrogen atom of -NHCO- is bonded to a carbon atom of the

benzene ring;

or its acid addition salt, which comprises reacting a compound represented by the following formula (II)

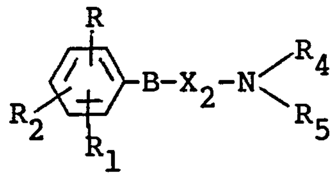 .....(II)

wherein $R_1$, $R_2$, $R_4$, $R_5$, $X_2$ and B are as defined with regard to formula (I), and R represents -OH or its alkali metal salt, or a halogen atom,

with a compound represented by the following formula (III)

$$R_3-X_1-Q \qquad .....(III)$$

wherein $R_3$ and $X_2$ are as defined, and Q represents a halogen atom or a sulfonic acid ester residue when R in formula (II) is -OH or its alkali metal salt, and -OH when R is a halogen atom.

57. A pharmaceutical composition comprising

(1) an antiarrhythmically effective amount of an aminoalkyl-substituted benzene derivative represented by the following formula

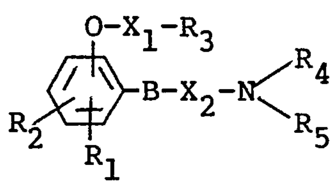 .....(I)

wherein $R_1$ represents a member selected from the class consisting of a hydrogen atom, lower alkyl groups, lower alkoxy groups, lower aliphatic acyl groups, halogen atoms and a trifluoroethoxy group; $R_2$ represents a hydrogen atom or a lower alkyl group; $R_3$ represents $-ONO_2$, $-CF_3$ or F; $R_4$ and $R_5$, independently from each other, represent a member selected from the class consisting of a

hydrogen atom, lower alkyl groups, halogen-substituted benzoyl groups, phenyl-substituted lower aliphatic acyl groups and a pyridinecarbonyl group, or $R_4$ and $R_5$ may form, together with the nitrogen atom to which they are bonded, a 6-membered hetero ring which may contain another hetero atom selected from O and N; $X_1$ and $X_2$, independently from each other, represents an alkylene group having 1 to 8 carbon atoms; and B represents -O- or -NHCO- in which the nitrogen atom of -NHCO- is bonded to a carbon atom of the benzene ring;

or a pharmaceutically acceptable acid addition salt thereof, and

(2) a pharmaceutically acceptable diluent or carrier.